(19) Europäisches Patentamt
European Patent Office
Office européen des brevets

(11) Publication number: **0 045 198**
**A2**

(12) **EUROPEAN PATENT APPLICATION**

(21) Application number: **81303410.5**

(22) Date of filing: **24.07.81**

(51) Int. Cl.³: **C 07 D 487/04**, A 61 K 31/40, C 07 D 205/08, C 07 D 405/12 // (C07D487/04, 209/00, 205/00)

(30) Priority: **24.07.80 PC /JP80/001 68**
**19.03.81 PC /JP81/000 60**

(43) Date of publication of application: **03.02.82**
**Bulletin 82/5**

(84) Designated Contracting States: **AT BE CH DE FR GB IT LI LU NL SE**

(71) Applicant: **Takeda Chemical Industries, Ltd., 27, Doshomachi 2-Chome, Higashi-ku Osaka, 541 (JP)**

(72) Inventor: **Morimoto, Akira, 7-8, Ueikeda 1-chome, Ikeda-shi Osaka 563 (JP)**
Inventor: **Miyawaki, Toshio, 17-27, Hinoikecho, Nishinomiya-shi Hyogo 662 (JP)**
Inventor: **Yoshioka, Kouichi, 5-7, Oharano-Kamisatotorimicho, Nishikyo-ku Kyoto-shi Kyoto 610-11 (JP)**

(74) Representative: **Laredo, Jack Joseph et al, Elkington and Fife High Holborn House 52/54 High Holborn, London, WC1V 6SH (GB)**

(54) **1,1-Disubstituted carba-2-penems and their production.**

(57) Novel carba-2-penem compounds having at the 1-position two substituents, at least one of which is other than an alkyl group, and represented by the formula

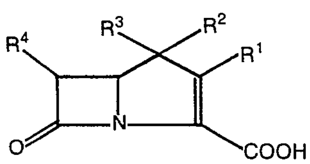

wherein $R^1$ is an alkyl, aryl, alkylthio, arylthio or acyl-aminoalkylthio group, $R^2$ and $R^3$ are each an alkoxycarbonyl or cyano group or a group of the formula $COR^1$ ($R^1$ being as above defined), $R^3$ may further be an alkyl or aryl group, and $R^4$ is a hydrogen atom, an alkyl, hydroxyalkyl or aryl group or an amino group which may optionally be protected, have excellent antibacterial and beta-lactamase inhibitory activities and are useful as drugs for use in humans and domestic animals.

EP 0 045 198 A2

1,1-Disubstituted Carba-2-penems and Their Production

This invention relates to novel carba-2-penem compounds, and salts and esters thereof, which have excellent antibacterial and beta-lactamase inhibitory activities, and intermediary compounds therefor, as well as a method of producing these compounds.

In the prior art, a number of "carba-2-penem" compounds having a fundamental skeleton represented by the formula

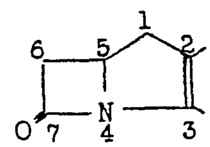

and having no substituents at the 1-position are known (e.g. Japanese Tokkyo Kokai Koho (published unexamined patent application) 78-87,390). Recent publications describe ones having one substituent at the 1-position (Japanese Tokkyo Kokai Koho 80-69,586, etc.) and ones having two methyl groups at the 1-position (Tetrahedron Letters, vol. 21, page 4009 (1980)). However, carba-2-penem compounds having two substituents at the the 1-position, at least one of which is other than an alkyl group are unknown.

According to a systematic nomenclature, the fundamental skeleton of the above carba-2-penem compounds should be named 7-oxo-1-azabicyclo[3.2.0]hept-2-ene,

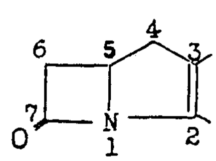

but herein, for simplification, it is named "carba-2-penem" as used above, which is derived from "penam" generally used in the field of penicillin chemistry.

This invention is concerned with novel carba-2-penem compounds having at the 1-position of the above-mentioned carba-2-penem skeleton two substituents, at least one of which is other than an alkyl group, and having the formula

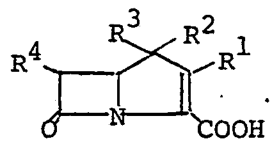

(I)

wherein $R^1$ is an alkyl, aryl, alkylthio, arylthio or acylaminoalkylthio group, $R^2$ and $R^3$ are each an alkoxycarbonyl or cyano group or a group of the formula $COR^1$ ($R^1$ being as above defined), $R^3$ may further be an alkyl or aryl group, and $R^4$ is a hydrogen atom, an alkyl, hydroxyalkyl or aryl group or an amino group which may be protected, and salts and esters thereof as well as a method of producing the same.

The present inventors have found that ring closure (cyclization) of compounds of the formula

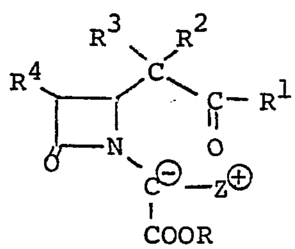

(II)

wherein $R^1$, $R^2$, $R^3$ and $R^4$ are as above defined, COOR is a protected carboxyl group, and Z is a phosphonio or phosphono group, leads to formation of carba-2-penem compounds of the formula

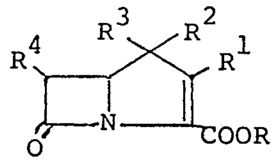

(III)

wherein the symbols have the same meanings as defined above, and that the thus-obtained carba-2-penem compounds (I) have excellent antibacterial and beta-lactamase inhibitory activities, and are used as therapeutic agents for infectious diseases caused by Gram-positive or negative bacteria in mammals including humans, dogs, cats, cattles, horses, mice and guinea pigs, as preservatives for feeds and industrial water, or as disinfectants for sanitary utensils, and furthermore as inhibitors of decomposition of beta-lactam antibiotics through combined use with said antibiotics, and have completed the present invention on the basis of these findings.

The above-mentioned alkyl group and the alkyl moiety of the hydroxyalkyl, alkylthio or acylaminoalkylthio group each includes straight or branches chain lower alkyl groups containing 1-6 carbon atoms, such as methyl, ethyl, n-propyl, isopropyl, n-butyl, isobutyl, sec-butyl, tert-butyl, n-pentyl and isopentyl. The aryl group and the aryl moiety of the arylthio group each includes phenyl and naphthyl, for instance. The acyl moiety of the acylaminoalkylthio includes lower aliphatic acyl groups containing 1-6 carbon atoms, such as formyl, acetyl, propionyl, isopropionyl, butyryl, and isobutyryl; aralkylcarbonyl groups, such as phenylacetyl and 3-phenylpropionyl; arylcarbonyl groups, such as benzoyl and so on. The alkoxy moiety of the alkoxycarbonyl group includes straight or branched lower alkoxy groups containing 1-6 carbon atoms, such as methoxy, ethoxy, n-propoxy, isopropoxy, n-butoxy, isobutoxy, sec-butoxy, tert-butoxy,

n-pentoxy and isopentoxy. The above-mentioned alkyl and aryl groups or moieties may be substituted. The substituted alkyl and aryl groups or moieties include, when the substituent is an alkoxy group such as mentioned above, alkoxyalkyl groups, such as methoxymethyl, 1-methoxyethyl, 2-methoxyethyl and 2-ethoxyethyl, and alkoxyaryl groups, such as o-, m- or p-methoxyphenyl and o-, m- or p-ethoxyphenyl, and, when the substituent is a halogen atom such as a fluorine, chlorine or bromine atom, haloalkyl groups, such as mono-, di- or trichloromethyl, mono-, di- or tribromomethyl, 2-mono-, di- or trifluoroethyl and 1-mono- or dichloroethyl, and haloaryl groups, such as o-, m- or p-chloro- or bromophenyl and 2,3-, 2,4- or 3,4-dichloro- or dibromophenyl. The aryl group or moiety may further be substituted by an alkyl group among those mentioned above. The alkyl-substituted aryl group is, for example, tolyl, xylyl or o-, m- or p-ethylphenyl. The protective group for the amino group includes such amino-protective groups in general use as alkylcarbonyl groups e.g. formyl, acetyl and propionyl; optionally substituted alkoxycarbonyl groups e.g. tert-butoxycarbonyl and trichloroethoxycarbonyl; alkoxyalkylcarbonyl groups e.g. methoxyacetyl and methoxypropionyl; substituted alkylcarbonyl groups e.g. monochloromethylcarbonyl, monochloroethylcarbonyl, dichloromethylcarbonyl, dichloroethylcarbonyl, trichloroethylcarbonyl and trichloropropylcarbonyl; aralkyloxycarbonyl groups e.g. benzyloxycarbonyl; substituted aralkyloxycarbonyl groups e.g. p-nitrobenzyloxycarbonyl; aralkyl groups e.g. benzyl, dibenzyl and trityl; and a tetrahydropyranyl group. The COOR represents a protected carboxyl group, and the protective group

R includes, for example, alkyl, haloalkyl, alkoxyalkyl, acyloxyalkyl, alkoxycarbonyloxyalkyl and aralkyl groups, a benzhydryl group and a phthalidyl group, etc. The alkyl, alkoxy, acyl and halo groups or moieties in said groups are, for example, as above mentioned, and the aralkyl groups include benzyl, phenethyl and tolubenzyl, which may optionally be substituted by nitro, hydroxyl, halogen, such alkyl or alkoxy as mentioned above, etc.

Favorable results are obtained especially when $R^1$ is, for example, $C_{1-4}$ lower alkyl, phenyl, $C_{1-4}$ lower alkylthio, phenylthio or acetylaminoalkylthio, when $R^2$ is, for example, $C_{1-4}$ lower alkoxycarbonyl, cyano or $C_{1-4}$ lower alkylcarbonyl, when $R^3$ is, for example, $C_{1-4}$ lower alkyl, phenyl, $C_{1-4}$ lower alkoxycarbonyl or $C_{1-4}$ lower alkylcarbonyl, and/or when $R^4$ is H, $C_{1-4}$ lower alkyl or phenyl.

The objective carba-2-penem compounds (I) are produced by ring closure of compounds (II). The group Z in the starting materials of formula (II) is one of phosphonio or phosphono groups familiar in Witting Condensation reaction, and particularly, it is a triaryl- (e.g. triphenyl-) or tri-lower alkyl- (e.g. tributyl-) phosphonio group or a phosphono group diesterified by lower alkyl (e.g. ethyl) groups. (When Z is a phosphono group, it further contains a strongly basic cation, especially a metal ion such as lithium, sodium or potassium ion.) In some cases, triphenylphosphonio is preferred as the group Z, and in some other cases, a diethylphosphono group containing an alkali metal ion (e.g. sodium ion) is preferred.

The ring closure is effected spontaneously, or in other words, during manufacture of the starting material, or for example, by heating at about 30°C to about 200°C, preferably at about 50°C to about 150°C.

The reaction is preferably carried out in an inert solvent, such as an aliphatic, alicyclic or aromatic hydrocarbon (e.g.

hexane, cyclohexane, benzene, toluene, xylene, mesitylene), a halogenated hydrocarbon (e.g. methylene chloride), an ether (e.g. diethyl ether), a lower alkylene glycol di-lower-alkyl ether (e.g. dimethoxyethane, diethylene glycol dimethyl ether), a cyclic ether (e.g. dioxane, tetrahydrofuran), a carboxylic acid amide (e.g. dimethylformamide), a di-lower-alkyl sulfoxide (e.g. dimethyl sulfoxide), a lower alkanol (e.g. methanol, ethanol, tert-butanol), or a mixture thereof, if necessary in an inert gaseous atmosphere (e.g. argon or nitrogen atmosphere). The thus-produced carba-2-penem compounds (III) can be isolated and purified by conventional methods such as concentration, acidity-basicity conversion, phase transfer, solvent extraction, crystallization, recrystallization and chromatography.

The protected carboxyl group at the 3-position of the thus-obtained compounds (III) can be converted to a free carboxyl group by a method known per se. Thus, for example, a halo-lower-alkyl protective group (e.g. 2,2-dibromoethyl, 2,2,2-trichloroethyl) as the group R can be removed with zinc-acetic acid, an aralkyl (e.g. benzyl, p-nitrobenzyl) or benzhydryl protective group by catalytic reduction with hydrogen-palladium on carbon or by treatment with an alkali metal sulfide (e.g. sodium sulfide, potassium sulfide), and an o-nitrobenzyl protective group by light irradiation.

The so-obtained carba-2-penem compounds (I), according to circumstances, may each have isomers due to steric configuration of substituents at the 1, 5 and 6 positions, and of groups in substituents at 6-position. When the compound (I) obtained is a mixture of isomers, it can be separated into individual isomers by a method known per se such as fractional crystallization or adsorption chromatography (column chromatography or thin layer chromatography)

or an other appropriate separation method. The racemic mixture may also be separated into individual antipodes in a conventional manner by introducing an adequate salt-forming group into the same, then forming a diastereomer salt mixture, for instance, and converting the optically active salt to a free compound, or by subjecting the salt mixture to fractional crystallization from an optically active solvent.

The carba-2-penem compounds (I) can be used not only in the free acid form but also after converting to the pharmacologically acceptable salt form in a manner known per se. The salt form includes alkali or alkaline earth metal salts such as lithium, sodium, potassium, calcium and magnesium salts and ammonium salts such as ammonium, cyclohexylammonium, diisopropylammonium and triethylammonium salts. Preferred are sodium and potassium salts.

The thus-obtained carba-2-penem compounds (I) and salts thereof are valuable antibiotics which are active against various Gram-positive and Gram-negative bacteria, and are usable as drugs for use in humans and domestic animals. Especially, they can be used safely as antibacterial agents in the treatment of infectious diseases caused by such Gram-positive or Gram-negative bacteria as *Staphylococcus aureus*, *Escherichia coli*, *Klebsiella pneumoniae*, etc. In addition, the antimicrobial agents of this invention are added to animal rations as disinfectants for preservation of feeds. The antibiotics can also be used in the form of an aqueous formulation having a concentration in the range of 0.1 to 100 ppm (i.e., 0.1 to 100 parts of the antibiotic per million parts of the solution) as antimicrobial preparations in order to destroy and inhibit growth of harmful bacteria, for example, on a medical and dental equipment or in order to inhibit growth of harmful bacterial life in an industrial

aqueous medium, for example, water-based points or paper mill white water.

The objective compounds (I) and salts and esters thereof of the present invention can be used alone or in combination with other active ingredients in some of various medicinal preparations in the form of capsules, tablets, powders, solutions, suspensions or elixirs, for instance. These can be administered orally, intravenously or intramuscularly.

The tablets for oral administration may contain usual excipients such as binders (e.g. syrup, gum arabic, gelatin, sorbitol, tragacanth gum, polyvinylpyrrolidone), fillers (e.g. lactose, sugars, corn starch, calcium phosphate, sorbitol, glycine), lubricants (e.g. magnesium stearate, talc, polyethylene glycol, silica) and disintegration promoting agents (e.g. potato starch, adequate wetting agents such as sodium laurylsulfate). The tablets may be coated by methods well known in the art. The liquid preparations for oral administration may be dried products which can be converted to aqueous or oleaginous suspensions, solutions, emulsions, syrups, elixirs and so on or which are to be dissolved in water or other adequate solvents prior to use. Such liquid preparations may contain suspending agents, such as sorbitol syrup, methylcellulose, glucose/sugar syrup, gelatin, hydroxyethylcellulose, carboxymethylcellulose, aluminum stearate gel, hydrogenated edible oils (e.g. almond oil, fractionated palm oil), oily esters, propylene glycol and ethanol, as well as preservatives, such as methyl or propyl p-hydroxybenzoate and sorbic acid. The suppositories may be prepared by using usual suppository bases, such as cocoa butter and other glycerides.

The injectable compositions may be provided in unit dosage forms in ampules or in containers with preservatives added thereto.

Said compositions may be in the form of suspensions, solutions or emulsions in oleaginous or aqueous solvents, and may contain as necessary such auxiliary agents as suspending agents, stabilizers and/or dispersing agents. The active ingredients may be supplied in the form of powders which are to be converted to injectable forms by using an adequate solvent, such as sterilized pyrogen-free water, prior to use.

They may also be made into preparations suitable for absorption by the mucous membrane of the nose and throat or by the bronchial tissue, such as powders, liquid sprays or inhalants, lozenges and laryngeal paints. For administration to the eye or ear, the agents may be used in the form of liquid- or semisolid-containing capsules or drops. Furthermore, they may be in the form of preparations for external application made by using hydrophobic or hydrophilic bases, such as ointments, creams, lotions, paints and powders.

The preparations may contain, in addition to the vehicles or carriers, such other components as stabilizers, binders, antioxidants, preservatives, lubricants, suspending agents, thickening agents and flavors. Furthermore, the compositions may contain other active ingredients for broadening the antibacterial spectrum.

For administration to domestic animals, they may be formulated as preparations for administration to the mammary gland using bases capable of allowing prolonged action or rapid release of the active ingredients.

The doses depend on the condition of the patient to be treated, the body weight of the host, the route and frequency of administration and the parenteral procedure suitable for general infections or oral procedure employed for intestinal infections.

Generally, the daily oral dose, in one or more applications a day, is about 15-600 mg of the active ingredient per kg of body weight of the patient. An adequate daily dose for human adults is within the range of about 80-120 mg of the active ingredient per kg of body weight of the patient.

The compositions of the invention may be administered in several unit dosage forms, such as solid or liquid unit dosage forms which can be taken orally. The lquid or solid unit dosage form compositions contain the active ingredients in contents of 0.5-99%. An adequate range is about 10-60%. Generally, the compositions contain about 15-1,500 mg of the active ingredients. However, preferable doses are generally within the range of about 250-1,000 mg.

In addition to the uses mentioned above, the compounds of the invention as well as salts and esters thereof have beta-lactamase inhibitory activity, and therefore can be used also in combination with beta-lactam type antibiotics. Examples of such beta-lactam type antibiotics are penicillins, such as benzylpenicillin, phenoxymethylpenicillin, carbenicillin, ampicillin, amoxicillin and sulbenicillin, and cephalosporins, such as cephaloridine, cephalothin, cefazolin, cephalexin, cefoxitin, cefacetrile, cefamandole, cefmenoxime, cefsulodin, cefotiam, cefotaxime, cefapirin, ceftizoxime, cefradine and cephaloglycine.

The starting compounds of formula (II) can be prepared, for example, according to the following reaction scheme.

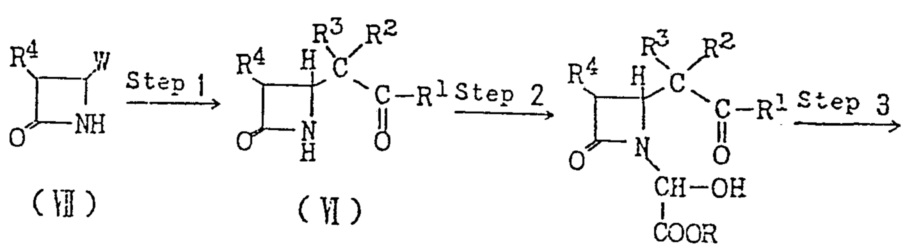


(VII) (VI) (V)

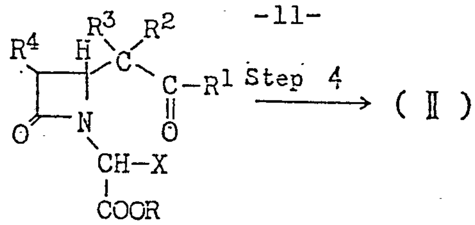


( IV )

In the above formulas, W is an acyloxy, sulfonyl or azido group or a halogen atom, X is a halogen atom or an organic sulfonyloxy group, and the remaining symbols have the same meanings as defined above.

Step 1

The azetidinone of formula (VI) is prepared by reacting a 4-W-azetidinone of formula (VII) with an active methyne compound of the formula

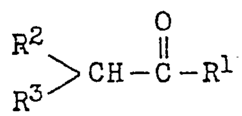

(VIII)

wherein $R^1$, $R^2$ and $R^3$ are as above defined, if desired followed by separation of the resulting mixture of isomers into individual isomers.

The symbol W in the starting material of formula (VII) represents, for example, an acyloxy group, a sulfonyl group $R^o-SO_2-$ ($R^o$ being an organic group), an azido group or a halogen atom. The acyl moiety of the acyloxy group W is a group derived from an organic carboxylic acid (including an optically active carboxylic acid), and has, for example, the same meaning as an acyl group $R^5-CO-$ wherein $R^5$ is, for example, a lower alkyl, cycloalkyl, cycloalkyl-lower-alkyl, phenyl or phenyl-lower-alkyl group, which may optionally be substituted. In the sulfonyl group $R^o-SO_2-$, $R^o$ is, for example, an aliphatic, aromatic-aliphatic or aromatic hydrocarbon residue having not more than 12 carbon atoms, which may optionally be substituted, and preferably a lower

alkyl group such as methyl or ethyl, a methyl group substituted by an optically active group, such as camphoryl. or benzyl, phenyl or tolyl. The halogen atom W includes a bromine atom, an iodine atom and preferably a chlorine atom. W is preferably an acetoxy group. This reaction is carried out in water, a water-miscible organic solvent or an organic solvent, preferably under basic conditions. As the base, there may be used, for example, an alkali or alkaline earth metal alcoholate (e.g. sodium methylate, sodium ethylate, potassium tert-butoxide), an alkali or alkaline earth metal hydride (e.g. sodium hydride, potassium hydride, calcium hydride), an alkali or alkaline earth metal hydroxide (e.g. sodium hydroxide, potassium hydroxide), an alkali or alkaline earth metal carbonate (e.g. potassium carbonate, sodium carbonate), n-butyllithium phenyllithium, lithium diisopropylamide, or 1,5-diazabicyclo[3.4.0]uncane. Usable organic solvents include, among others, lower alcohols (e.g. methanol, ethanol), ketones (e.g. acetone), esters (e.g. ethyl acetate), ethers (e.g. ethyl ether, tetrahydrofuran, dioxane), and amides (e.g. dimethylformamide). The reaction is carried out at -80°C to room temperature, and may also be carried out at somewhat higher temperatures. The thus-produced compound (VI) may be isolated and purified by a known method such as mentioned hereinbefore. The reaction mixture containing the compound (VI) as it is may also be used as a raw material for the next reaction.

According to circumstances, the compound of formula (VI) may be obtained as a mixture of optically inactive isomers. Separation of the isomers is carried out by a usual method, particularly by chromatography and/or crystallization.

Those starting materials of formula (VII) in which $R^4$ is

a hydrogen atom or an alkyl or aryl group are known compounds and can be prepared by the method of Clauss et al. (Justus Liebigs Annalen der Chemie, <u>1974</u>, p. 589).

Those compounds in which $R^4$ is an amino group which may optionally be substituted are also known and can be prepared by the method of Brain et al. (Journal of Chemical Society Perkin I, <u>1975</u>, p. 562).

Those compounds in which $R^4$ is a hydroxyalkyl group ($R^5R^6\overset{OH}{C}-$) can be prepared according to the following reaction scheme.

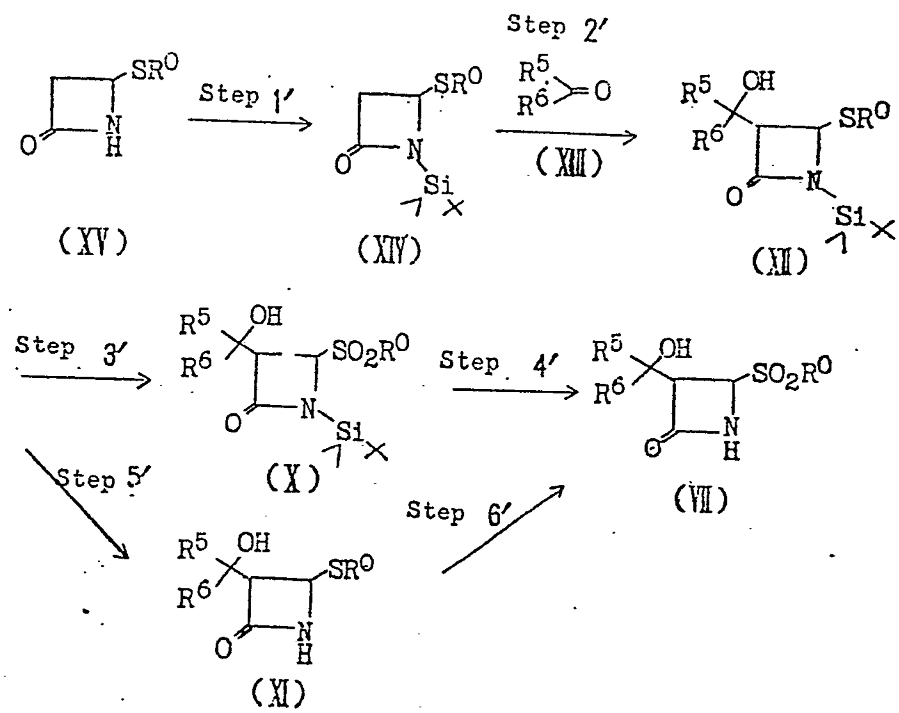


(XV) (XIV) (XIII) (XII) (X) (VII) (XI)

In the above formulas, $R^0$ is as above defined, and $R^5$ and $R^6$ are each a hydrogen atom or an aliphatic, aromatic or aromatic-aliphatic hydrocarbon residue.

<u>Step 1'</u>

The compound of formula (XIV) is prepared by reacting an azetidinone of formula (XV) with tert-butyldimethylchlorosilane.

The symbol $R^0$ for the compound of formula (XV) represents,

for example, an aliphatic, aromatic-aliphatic or aromatic hydrocarbon residue containing not more than 12 carbon atoms, which may optionally be substituted, and preferably a lower alkyl group such as methyl or ethyl, a methyl group substituted by an optically active group, such as camphoryl, or benzyl, phenyl or tolyl. This reaction is carried out in an organic solvent, preferably under basic conditions. As the base, there may be used triethylamine or tri-n-butylamine, for instance. The organic solvent includes esters (e.g. ethyl acetate), ethers (e.g. ethyl ether, tetrahydrofuran, dioxane), ketones (e.g. acetone), and amides (e.g. dimethylformamide).

Step 2'

The compound of formula (XII) is prepared by reacting a compound of formula (XIV) with a compound of the formula

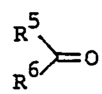 (XIII)

wherein $R^5$ and $R^6$ are as above defined, in the presence of a base, if desired followed by separation of the resulting mixture of isomers into individual isomers.

$R^o$ in formula (XIV) has the same meaning as defined above. This reaction is carried out in an organic solvent in the presence of a base. The base is, for example, hydride (e.g. sodium hydride, potassium hydride, calcium hydride), n-butyllithium, phenyllithium or lithium diisopropylamide. Usable solvents are, for example, ethers (e.g. ethyl ether, tetrahydrofuran, dioxane). The reaction is carried out at -80°C to room temperature, preferably at -80°C to -30°C.

Step 3'

The compound of formula (X) is prepared by reacting a

compound of formula (XII) with an oxidizing agent.

$R^o$, $R^5$ and $R^6$ in formula (XII) have the same meanings as defined above. This reaction is carried out in water, a water-miscible organic solvent or an organic solvent in the presence of an oxidizing agent. The oxidizing agent is, for example, potassium permanganate, periodic acid, or an organic peracid (e.g. peracetic acid, pertrifluoroacetic acid, perbenzoic acid, m-chloroperbenzoic acid). Usable organic solvents include, among others, lower alcohols (e.g. methanol, ethanol), esters (e.g. ethyl acetate), ethers (e.g. ethyl ether, tetrahydrofuran, dioxane), halogenated hydrocarbons (e.g. chloroform, dichloromethane) and amides (e.g. dimethylformamide). The reaction is preferably carried out under ice-cooling or at room temperature.

Step 4'

The sulfone of formula (VII) is prepared by subjecting a compound of formula (X) to hydrolysis in the presence of an acid or a fluoride.

This reaction is carried out in water, a water-miscible organic solvent or an organic solvent. Usable solvents include lower alcohols (e.g. methanol, ethanol), ketones (e.g. acetone), ethers (e.g. tetrahydrofuran, dioxane) and amides (e.g. dimethylformamide). The acid is, for example, hydrochloric acid or an organic acid (e.g. acetic acid). The fluoride is, for example, tetra-n-butylammonium fluoride. The reaction is preferably carried out under ice cooling or at room temperature.

Step 5'

The compound of formula (XI) is prepared by hydrolyzing a compound of formula (XII) in the presence of an acid or a fluoride.

This reaction is carried out in water, a water-miscible organic solvent or an organic solvent. Usable organic solvents

include lower alcohols (e.g. methanol, ethanol), ketones (e.g. acetone), ethers (e.g. tetrahydrofuran, dioxane) and amides (e.g. dimethylformamide). The acid is, for example, hydrochloric acid or an organic acid (e.g. acetic acid). The fluoride is, for example, tetra-n-butylammonium fluoride. The reaction is preferably carried out under ice-cooling or at room temperature.

<u>Step 6'</u>

The sulfone of formula (VII) is prepared by reacting a compound of formula (XI) with an oxidizing agent.

$R^o$, $R^5$ and $R^6$ in formula (XI) have the same meanings as defined above. This reaction is carried out in water, a water miscible organic solvent or an organic solvent in the presence of an oxidizing agent. The oxidizing agent is, for example, potassium permanganate, periodic acid or an organic peracid (e.g. peracetic acid, pertrifluoroacetic acid, perbenzoic acid, m-chloroperbenzoic acid). Usable organic solvents include, among others, lower alcohols (e.g. methanol, ethanol), esters (e.g. ethyl acetate), ethers (e.g. ethyl ether, tetrahydrofuran, dioxane), halogenated hydrocarbons (e.g. chloroform, dichloromethane) and amides (e.g. dimethylformamide). The reaction is preferably carried out under ice cooling or at room temperature.

<u>Step 2</u>

The compound of formula (V) is prepared by reacting a glyoxylic acid of the formula

$$OHC-\overset{\overset{\displaystyle O}{\|}}{C}-OR \qquad (IX)$$

wherein COOR is as above defined, or an appropriate derivative thereof, such as a hydrate or a hemiacetal (e.g. with a lower alkanol such as methanol or ethanol), with a

compound of formula (VI), if desired followed by separation of the thus-obtained mixture of isomers into individual isomers. The addition of the glyoxylic acid ester compound to the nitrogen atom in the lactam ring is carried out at room temperature or if desired under heating at about 100°C, for instance. When a hydrate of a glyoxylic acid compound is used, water is formed, which is removed if desired by distillation, such as azeotropic distillation, or by an adequate dehydration method such as the use of molecular sieves. The above reaction is preferably carried out in the presence of an adequate solvent, such as dioxane, toluene or dimethylformamide or a mixture of these, in an inert gas atmosphere, such as a nitrogen atmosphere. The reaction mixture as it is may be used as a raw material in the next step, although the compound (V) thus produced may be isolated and purified by a known method such as mentioned above.

Step 3

The compound of formula (IV) wherein X is a halogen atom or a reactive ester residue, preferably an organic sulfonyloxy group, is prepared by converting the secondary hydroxyl group of a compound of formula (V) to a halogen atom, such as a chlorine or bromine atom, or an organic sulfonyloxy, such as a lower alkylsulfonyloxy (e.g. methylsulfonyloxy) or an arylsulfonyloxy (e.g. 4-methylphenylsulfonyloxy), and, if desired, the thus-obtained mixture of isomers may be separated into individual isomers.

This reaction is carried out by using a halogenating agent, such as a thionyl halide (e.g. chloride) or a phosphorus oxyhalide (e.g. chloride), or an appropriate esterifying agent, such as

a halophosphonium halide (e.g. triphenylphosphine dibromide or diiodide) or an appropriate organic sulfonic acid halide (e.g. chloride), preferably in the presence of a base, preferably an organic base, such as an aliphatic tertiary amine (e.g. triethylamine, diisopropylethylamine), polystyrene-Hünig base or a pyridine-type heterocyclic base (e.g. pyridine or colidine). The above reaction is preferably carried out in an appropriate solvent, such as dioxane, tetrahydrofuran or a mixture of these, if desired under cooling and/or in an inert gas atmosphere, such as nitrogen atmosphere.

The halogen atom or reactive ester residue X in the thus-prepared compound of formula (IV) may be converted to another halogen atom or reactive ester residue by a method known per se. For example, the chlorine atom may be replaced by an iodine atom by treating a corresponding chlorine compound with an appropriate iodide, such as sodium iodide, preferably in the presence of an adequate solvent such as dimethylformamide. The reaction mixture containing the so-prepared compound (IV) may be used as it is as a raw material in the next step, although the compound (IV) may be isolated and purified by a known method such as mentioned above.

Any of optically inactive pure isomers of formula (IV) and mixtures thereof may be used in the above-mentioned reaction.

Step 4

The starting material of formula (II) is prepared by reacting a compound of formula (IV), wherein X is a halogen atom or a reactive ester residue, with an appropriate phosphine compound, such as a tri-lower-alkylphosphine (e.g. tri-n-butylphosphine), a tri-arylphosphine (e.g. tri-phenylphosphine), or an

appropriate phosphite compound, such as a tri-lower-alkylphosphite (e.g. triethyl phosphite), or an alkali metal dimethyl phosphite.

The above reaction is preferably carried out in the presence of an appropriate inert solvent, such as a hydrocarbon (e.g. hexane, cyclohexane, benzene, toluene or xylene), an ether (e.g. dioxane, tetrahydrofuran or diethylene glycol dimethyl ether) or an amide (e.g. dimethylformamide), or a mixture of these. The above reaction is conducted, depending on the reactivity, under cooling or at raised temperatures, namely at about -10°C to 100°C, preferably at about 20°C to 80°C, and/or in an inert gas atmosphere, such as a nitrogen atmosphere. For preventing possible oxidation reactions, a catalytic amount of an antioxidant, such as hydroquinone, may be added.

When a phosphine compound is used, the above reaction is usually carried out in the presence of a base, such as an organic base, for example an amine (e.g. triethylamine, diisopropylethylamine, 2,6-lutidine or polystyrene-Hünig base). The reaction mixture containing the thus-produced compound of formula (II) may be used as it is as a starting material for the reaction in accordance with the present invention, although the compound (II) may be isolated and purified by a known method such as mentioned above.

Any of optically inactive pure isomers of formula (II), mixtures thereof and corresponding optically active isomers may be used in the next reaction step.

Example 1

In 50 ml of tetrahydrofuran, there are dissolved 6.5 g of 4-acetoxy-2-azetidinone and 5.7 g of 2,4-dioxo-3-methylpentane, and the solution is cooled to -5°C and stirred. Thereto is added dropwise 50 ml of 1N aqueous sodium hydroxide solution. After the dropping, the mixture is stirred at -5°C for 15 minutes, and then extracted with ethyl acetate. The organic layer is washed with a saturated aqueous sodium chloride solution and dried over $Na_2SO_4$, and the solvent is distilled off. The residue is purified by passing it through a silica gel column. Elution with chloroform gives 3.25 g of 4-(1,1-diacetylethyl)-2-azetidinone.

Melting point: 72-74°C

Elemental analysis

Calculated for $C_9H_{13}NO_3$: C 59.00; H 7.15; N 7.65

Found: C 58.81; H 7.13; N 7.49

Infrared absorption spectrum (KBr): 1690cm$^{-1}$, 1715cm$^{-1}$, 1750cm$^{-1}$

NMR spectrum (60 MHz, in $CDCl_3$): $\delta$ 1.37 (3H, singlet), 2.20 (6H, singlet), 2.46 - 3.34 (2H, multiplet), 4.38 (1H, doublet of doublet), 7.00 (1H, multiplet)

Example 2

A suspension of 0.8 g of sodium hydride (60% in oil) in 20 ml of tetrahydrofuran is stirred with ice cooling. Thereto is added 2.6 g of 2,4-dioxo-3-ethylpentane, the mixture is stirred for 10 minutes and then cooled to -10°C, and 2.6 g of 4-acetoxy-2-azetidinone is added. After stirring for 15 minutes, the

reaction mixture is poured into a saturated aqueous solution of sodium chlroide, and extracted with ethyl acetate. The organic layer is washed with a saturated aqueous solution of sodium chloride and dried over $Na_2SO_4$. The solvent is distilled off under reduced pressure, the residue is treated with isopropyl ether, and the resulting crystals are collected by filtration and dried to give 2.88 g of 4-(1,1-diacetylpropyl)-2-azetidinone.

Melting point: 110-112°C

Elemental analysis

Calculated for $C_{10}H_{15}NO_3$: C 60.89; H 7.67; N 7.10

Found: C 61.02; H 7.58; N 7.12

Infrared absorption spectrum (KBr): 1685cm$^{-1}$, 1710cm$^{-1}$, 1760cm$^{-1}$

NMR spectrum (60 MHz, in $CDCl_3$): $\delta$ 0.90 (3H, triplet), 2.05 (2H, quartet), 2.20 (3H, singlet), 2.21 (3H, singlet), 2.54-3.37 (2H, multiplet), 4.26 (1H, doublet of doublet), 6.32 (1H, multiplet)

Example 3

Diethyl acetylmalonate (2.05 g) and 1.30 g of 4-acetoxy-2-azetidinone are dissolved in 20 ml of tetrahydrofuran, and the solution is cooled to -5°C and stirred. Thereto is added dropwise 10 ml of 1N aqueous sodium hydroxide solution. Thereafter, the mixture is stirred at room temperature for 15 hours. The reaction mixture is extracted with ethyl acetate, and the organic layer is washed with a saturated aqueous sodium chloride solution and dried over $Na_2SO_4$. The solvent is distilled off under reduced pressure, and the residue is purified by passing it through

a silica gel column. Elution with chloroform gives 0.86 g of 4-(1,1-diethoxycarbonyl-2-oxopropyl)-2-azetidinone as an oil.
NMR spectrum (60 MHz, in $CDCl_3$): δ 1.32 (6H, triplet), 2.35 (3H, singlet), 3.15 (2H, doublet of doublet), 4.07 – 4.50 (1H, multiplet), 4.34 (4H, quartet), 6.61 (1H, multiplet)

Example 4

Ethyl 2-acetylpropionate (4.32 g) and 3.87 g of 4-acetoxy-2-azetidinone are dissolved in 30 ml of tetrahydrofuran, and the solution is stirred at -10°C with cooling. Thereto is added dropwise 30 ml of 1N aqueous sodium hydroxide solution. Thereafter, the reaction is allowed to proceed for 2 hours. The reaction mixture is extracted with ethyl acetate, and the organic layer is washed with a saturated aqueous sodium chloride solution and dried ($MgSO_4$). The solvent is distilled off under reduced pressure and the residue is purified by passing it through a silica gel column. Elution with chloroform gives 5.11 g of 4-(1-acetyl-1-ethoxycarbonyl-ethyl)-2-azetidinone as an oil.
Infrared absorption spectrum (liq.): 1705 $cm^{-1}$, 1750 $cm^{-1}$
NMR spectrum (60 MHz, in $CDCl_3$): δ 1.26 (3H, triplet), 1.36 (3H, singlet), 2.17 (3H, singlet), 2.60 – 3.25 (2H, multiplet), 4.17 (1H, multiplet), 4.28 (2H, quartet), 6.96 (1H, multiplet)

Example 5

1-Cyano-1-phenyl-2-oxopropane (0.796 g) is dissolved in 20 ml of tetrahydrofuran, 0.76 g of 1,8-diazabicyclo[5.4.0]-7-undecene is added, and the mixture is stirred for 5 minutes and cooled to -10°C. Then 0.65 g of 4-acetoxy-2-azetidinone is added, and the reaction is allowed to proceed for 15 minutes and then at room temperature for further 30 minutes. The reaction mixture is poured into a saturated aqueous sodium chloride solution and extracted with ethyl acetate. The organic layer is washed with diluted hydrochloric acid and then with a saturated aqueous sodium chloride solution, and dried ($Na_2SO_4$), and the solvent is distilled off under reduced pressure. The residue is passed through a silica gel column. Elution with chloroform gives 0.473 g of 4-(1-cyano-1-phenyl-2-oxopropyl)-2-azetidinone as an oil.

NMR spectrum (60 MHz, in $CDCl_3$): $\delta$ 2.30 (3H, singlet), 2.76 - 3.25 (2H, multiplet), 4.35 (1H, multiplet), 5.80 ($\frac{1}{2}$H, multiplet), 6.24 ($\frac{1}{2}$H, multiplet), 7.36 (5H, singlet)

Crystallization of the above oil from an ether-n-hexane mixed solvent gives one of the isomers.

Melting point: 115-121°C

Elemental analysis

Calculated for $C_{13}H_{12}N_2O_2$: C 68.40; H 5.30; N 12.28

Found: C 68.19; H 5.25; N 12.14

Infrared absorption spectrum (KBr): 1710cm$^{-1}$, 1725cm$^{-1}$, 1750cm$^{-1}$

NMR spectrum (60 MHz, in $CDCl_3$): $\delta$ 2.30 (3H, singlet), 2.86 (2H, doublet of doublet), 4.38 (1H, triplet), 6.34 (1H, multiplet), 7.50 (5H, singlet)

Example 6

Triacetylmethane (0.72 g) is dissolved in 10 ml of tetrahydrofuran, and the solution is stirred at room temperature. Thereto is added 0.76 g of 1,8-diazabicyclo[5.4.0]-7-undecene. After stirring for 10 minutes, 0.65 g of 4-acetoxy-2-azetidinone is added, and the mixture is stirred at room temperature for 5 hours. The reaction mixture is poured into a saturated aqueous sodium chloride solution, adjusted to pH 5.0 with phosphoric acid, and extracted with ethyl acetate. The organic layer is washed with a saturated aqueous sodium chloride solution and then dried ($Na_2SO_4$), and the solvent is distilled off under reduced pressure. The residue is allowed to pass through a silica gel column. Elution with chloroform gives 57 mg of 4-(triacetylmethyl)-2-azetidinone as an oil. This turns into a crystalline form upon treatment with isopropyl ether.

Melting point: 82-84°C

Elemental analysis

Calculated for $C_{10}H_{13}NO_4$: C 56.86; H 6.20; N 6.63

Found: C 56.73; H 6.13; N 6.28

Infrared absorption spectrum (KBr): 1695cm$^{-1}$, 1725cm$^{-1}$, 1763cm$^{-1}$

NMR spectrum (60 MHz, in $CDCl_3$): $\delta$ 2.29 (9H, singlet), 2.74 – 3.31 (2H, multiplet), 4.54 (1H, doublet of doublet), 6.63 (1H, multiplet)

Example 7

A suspension of 0.4 g of sodium hydride (60% in oil) in 15 ml of tetrahydrofuran is prepared and stirred with ice cooling. Thereto is added 1.95 g of methyl 2-benzoylpropionate. The mixture is stirred for 10 minutes and then cooled to -10°C. Then, 1.3 g of 4-acetoxy-2-azetidinone is added, and the mixture is

stirred at -10°C to -5°C for 30 minutes. The reaction mixture is poured into a saturated aqueous sodium chloride solution, and extracted with ethyl acetate, and the organic layer is washed with a saturated aqueous sodium chloride solution and dried ($Na_2SO_4$). The solvent is distilled off under reduced pressure, and the residue is allowed to pass through a silica gel column. Elution with chloroform gives an oil. This is crystallized from isopropyl ether to give 2.48 g of 4-(1-benzoyl-1-methoxycarbonylethyl)-2-azetidinone (1:6 mixture of isomers) as crystals.

Melting point: 90-92°C

Elemental analysis

Calculated for $C_{14}H_{15}NO_4$: C 64.35; H 5.79; N 5.36

Found: C 64.40; H 5.83; N 5.20

Infrared absorption spectrum (KBr): 1675cm$^{-1}$, 1725cm$^{-1}$, 1770cm$^{-1}$

NMR spectrum (60 MHz, in $CDCl_3$): $\delta$ 1.56 (3H × $\frac{1}{7}$, singlet), 1.60 (3H × $\frac{6}{7}$, singlet), 2.94 (2H, multiplet), 3.68 (3H × $\frac{6}{7}$, singlet), 3.70 (3H × $\frac{1}{7}$, singlet), 4.20 (1H, multiplet), 6.70 (1H, multiplet), 7.35 – 8.00 (5H, multiplet)

Example 8

4-Acetoxy-2-azetidinone (3.88 g) and 7.93 g of diethyl benzoylmalonate are dissolved in 30 ml of tetrahydrofuran, and the solution is stirred with ice cooling. Thereto is added 30 ml of 1N aqueous sodium hydroxide solution. The mixture is stirred for 30 minutes, and then at room temperature for further 3 hours. The reaction mixture is poured into a saturated aqueous sodium chloride solution, and extracted with ethyl acetate. The organic layer is washed with a saturated aqueous sodium chloride solution,

and dried ($Na_2SO_4$), and the solvent is distilled off under reduced pressure. The residue is purified by allowing to pass through a silica gel column. Elution with chloroform gives an oil. This is crystallized from ether-isopropyl ether to give 2.56 g of 4-(1-benzoyl-1,1-diethoxycarbonylmethyl)-2-azetidinone.

Melting point: 80-81°C

Elemental analysis

Calculated for $C_{17}H_{19}NO_6$: C 61.25; H 5.72; N 4.20

Found: C 61.12; H 5.69; N 4.32

Infrared absorption spectrum (KBr): 1690cm$^{-1}$, 1720cm$^{-1}$, 1750cm$^{-1}$

NMR spectrum (60 MHz, in $CDCl_3$): δ 1.12 (3H, triplet), 1.22 (3H, triplet), 3.27 (2H, multiplet), 4.27 (2H, quartet), 4.37 (2H, quartet), 4.66 (1H, doublet of doublet), 6.46 (1H, multiplet), 7.45-8.10 (5H, multiplet)

Example 9

A suspension of 0.4 g of sodium hydride (60% in oil) in 50 ml of tetrahydrofuran is prepared and stirred. Thereto is added with ice cooling 1.76 g of methyl 2-ethylthiocarbonylpropionate. The mixture is stirred for 10 minutes and then cooled to -10°C, and 1.29 g of 4-acetoxy-2-azetidinone is added thereto. After stirring for 40 minutes, the reaction mixture is poured into a saturated aqueous sodium chloride solution, and extracted with ethyl acetate. The organic layer is washed with a saturated aqueous sodium chloride solution and dried ($MgSO_4$), and the solvent is distilled off under reduced pressure. The residue is allowed to pass through a silica gel column. Elution with chloroform gives 1.31 g of 4-(1-ethylthiocarbonyl-1-methoxycarbonylethyl)-2-azetidinone as an oil.

Infrared absorption spectrum (liquid): $1760 cm^{-1}$, $1720 cm^{-1}$

NMR spectrum (60 MHz, in $CDCl_3$): $\delta$ 1.25 (3H, triplet), 1.50 (3H, singlet), 2.74 – 3.11 (4H, multiplet), 3.75 (3H, singlet), 4.30 (1H, multiplet), 7.04 (1H, multiplet)

Example 10

A suspension of 0.08 g of sodium hydride (60% in oil) in 10 ml of tetrahydrofuran is prepared and stirred with ice cooling. Thereto is added 260 mg of 2,4-dioxo-3-ethylpentane. After stirring for 10 minutes, the mixture is cooled to -10°C, and then 260 mg of 4-phenylsulfonyl-2-azetidinone is added thereto. The mixture is stirred at the same temperature for 5 minutes, then at room temperature for 10 minutes, poured into a saturated aqueous sodium chloride solution, and extracted with ethyl acetate. The organic layer is washed with a saturated aqueous sodium chloride solution and dried ($Na_2SO_4$), and the solvent is then distilled off under reduced pressure. The residue is allowed to pass through a silica gel column. Elution with chloroform gives an oil. This is crystallized from isopropyl ether to give 128 g of 4-(1,1-diacetylpropyl)-2-azetidinone.

Comparison of infrared absorption spectra and nuclear magnetic resonance spectra reveals identity of this product with that obtained in Example 2.

Example 11

4-Acetoxy-2-azetidinone (1.3 g) and 1.15 g of 2,4-dioxo-3-methylpentane are dissolved in 20 ml of acetone, 1.40 g of potassium carbonate is added, and the mixture is stirred at room temperature for 6 days. The insoluble matter is filtered off, and the filtrate is

concentrated under reduced pressure. The residue is allowed to pass through a silica gel column. Elution with chloroform gives 1.21 g of 4-(1,1-diacetylethyl)-2-azetidinone.

Comparison of infrared absorption spectra and nuclear magnetic resonance spectra reveals identity of this product with that obtained in Example 1.

Reference Example 1

4-Phenylthio-2-azetidinone (360 mg) is dissolved in 10 ml of dichloromethane, and the solution is stirred with ice cooling. Thereto are added 0.34 ml of triethylamine and 330 mg of tert-butyldimethylchlorosilane. The mixture is stirred with ice cooling for 7 hours. The reaction mixture is poured into a saturated aqueous sodium chloride solution, and extracted with ethyl acetate. The organic layer is dried ($Na_2SO_4$), and the solvent is distilled off under reduced pressure. The residue is allowed to pass through a silica gel column. Elution with chloroform gives 525 mg of 1-(tert-butyldimethylsilyl)-4-phenylthio-2-azetidinone as an oil.

NMR spectrum (60MHz, in $CDCl_3$): $\delta$ 0.30 (6H, singlet), 0.98 (9H, singlet), 3.20 (2H, doublet of AB quartet), 4.85 (1H, doublet of doublet), 7.25 (5H, singlet)

Reference Example 2

4-Ethylthio-2-azetidinone (1.31 g) and 1.1 g of triethylamine are dissolved in 10 ml of dimethylformamide, and the solution is stirred with ice cooling. Thereto is added 1.65 g of tert-butyldimethylchlorosilane, and the mixture is stirred at room temperature for 4 hours. The reaction mixture is poured into 1M aqueous solution of monopotassium phosphate, and extracted with

chloroform. The organic layer is washed with water and dried ($MgSO_4$), and the solvent is distilled off under reduced pressure. The residue is allowed to pass through a silica gel column. Elution with a 2:8 mixture of ethyl acetate and n-hexane gives 1.51 g of 1-(tert-butyldimethylsilyl)-4-ethylthio-2-azetidinone as an oil.

NMR spectrum (60MHz, in $CDCl_3$) : $\delta$ 0.26 (6H, singlet), 0.98 (9H, singlet), 1.27 (3H, triplet), 2.65 (2H, quartet), 3.30 (2H, doublet of AB quartet), 4.70 (1H, doublet of doublet)

Reference Example 3

Dried tetrahydrofuran (10 ml) is cooled to -70°C and stirred in a nitrogen atmosphere. Thereto are added in sequence 6.43 ml of n-butyllithium (15% n-hexane solution) and 1.44 ml of diisopropylamine, and the mixture is stirred for 10 minutes. Then, a solution of 1.51 g of 1-(tert-butyldimethylsilyl)-4-phenylthio-2-azetidinone in 5 ml of dried tetrahydrofuran is added, and the reaction is allowed to proceed for 25 minutes. After 0.9 ml of acetone is added dropwise, the reaction is allowed to proceed at -70°C for further 25 minutes. 10 ml of a saturated aqueous sodium chloride solution is added and the mixture is extracted with ethyl acetate. The organic layer is washed with a saturated aqueous sodium chloride solution and dried ($Na_2SO_4$), and the solvent is distilled off under reduced pressure. The residue is allowed to pass through a silica gel column. Elution with chloroform gives 1.527 g of 1-(tert-butyldimethylsilyl)-3-(1-hydroxy-1-methylethyl)-4-phenylthio-2-azetidinone as an oil.

NMR spectrum (60MHz, in $CDCl_3$): $\delta$ 0.32 (6H, singlet), 1.00 (9H, singlet), 1.13 (3H, singlet), 1.28 (3H, singlet), 2.40 (1H, singlet), 3.14 (1H, doublet), 4.88 (1H, doublet), 7.20–7.50 (5H, multiplet)

Refernce Example 4

Diisopropylamine (1.39 ml) is dissolved in 20 ml of dried tetrahydrofuran, and the solution is cooled to -70°C in a nitrogen atmosphere and stirred. Thereto is added 6.25 ml of n-butyllithium (15% n-hexane solution), and the mixture is stirred for 10 minutes. To the mixture is then added a solution of 1.22 g of 1-(tert-butyldimethylsilyl)-4-ethylthio-2-azetidinone in 5 ml of dried tetrahydrofuran, and the reaction is allowed to proceed for 20 minutes. Then, the reaction mixture is poured into ethyl acetate, and the mixture is washed with water and dried ($MgSO_4$) and the solvent is distilled off under reduced pressure to give 1.58 g of 1-(tert-butyldimethylsilyl)-4-ethylthio-3-(1-hydroxy-1-methylethyl)-2-azetidinone as an oil.

NMR spectrum (60 MHz, in $CDCl_3$): $\delta$ 0.32 (6H, singlet), 0.98 (9H, singlet), 1.30 (3H, singlet), 1.36 (3H, singlet), 2.54 (2H, quartet), 3.25 (1H, doublet), 4.66 (1H, doublet)

Reference Example 5

1-(tert-Butyldimethylsilyl)-3-(1-hydroxy-1-methylethyl)-4-phenylthio-2-azetidinone (71 mg) is dissolved in 5 ml of dichloromethane, and the solution is stirred with ice cooling. Thereto is added 140 mg of m-chloroperbenzoic acid, and the mixture is stirred for 30 minutes. Ethyl acetate is added to

the reaction mixture, the mixture is washed with a saturated aqueous sodium hydrogen carbonate solution and then with water, and dried ($Na_2SO_4$), and the solvent is distilled off under reduced pressurre. The residue is allowed to pass through a silica gel column. Elution with a 4:1 mixture of chloroform and ethyl acetate gives an oil. This is crystallized from n-hexane to give 36 mg of 1-(tert-butyldimethylsilyl)-3-(1-hydroxy-1-methylethyl)-4-phenylsulfonyl-2-azetidinone as white crystals.

Melting point: 147-150°C

Elemental analysis

Calculated for $C_{18}H_{29}NO_4SSi$: C 56.36; H 7.21; N 3.65

Found: C 56.29; H 7.57; N 3.75

Infrared absorption spectrum (KBr): $1755cm^{-1}$

NMR spectrum (60 MHz, in $CDCl_3$): δ 0.34 (6H, singlet), 0.74 (3H singlet), 1.01 (9H, singlet), 1.22 (3H, singlet), 3.26 (1H, doublet, 4.65 (1H, doublet), 7.27 – 8.05 (5H, multiplet)

Reference Example 6

1-(tert-Butyldimethylsilyl)-4-ethylthio-3-(1-hydroxy-1-methylethyl)-2-azetidinone (1.50 g) is dissolved in 40 ml of dichloromethane, and the solution is stirred at room temperature. Thereto is added 2.15 g of m-chloroperbenzoic acid, and the mixture is stirred for 20 minutes. The reaction mixture is washed with a saturated aqueous sodium hydrogen carbonate solution and with water, and dried ($MgSO_4$). The solvent is distilled off under reduced pressure to give 1.779 g of 1-(tert-butyldimethylsilyl)-4-ethylsulfonyl-3-(1-hydroxy-1-methylethyl)-2-azetidinone as an

oil.

NMR spectrum (60 MHz, in $CDCl_3$): δ 0.34 (6H, singlet), 1.00 (9H, singlet), 1.35 (9H, singlet), 1.40 (3H, triplet), 3.00 (2H, triplet), 3.46 (1H, doublet), 4.55 (1H, doublet)

Reference Example 7

1-(tert-Butyldimethylsilyl)-3-(1-hydroxy-1-methylethyl)-4-phenylsulfonyl-2-azetidinone (150 mg) is dissolved in a mixture of 9 ml of methanol and 1 ml of water, 0.15 ml of concentrated hydrochloric acid is added, and the mixture is stirred at room temperature for 3.5 hours. The reaction mixture is extracted with ethyl acetate, and the organic layer is washed with water and dried ($Na_2SO_4$). The solvent is distilled off under reduced pressure, and the residue is allowed to pass through a silica gel column. Elution with a 2:3 mixture of chloroform and ethyl acetate and then with ethyl acetate gives 58 mg of an oil. This is crystallized from isopropyl ether to give 35.5 mg of 3-(1-hydroxy-1-methylethyl)-4-phenylsulfonyl-2-azetidinone.

Melting point: 155-157°C

Elemental analysis

Calculated for $C_{12}H_{15}NO_4S$: C 53.51; H 5.61; N 5.20

Found: C 53.61; H 5.44; N 5.30

Infrared absorption spectrum (KBr): 1765cm$^{-1}$

NMR spectrum (90 MHz, in $d_6$-DMSO+$D_2O$): δ 1.14 (3H, singlet), 1.28 (3H, singlet), 3.28 (1H, doublet), 4.90 (1H, doublet), 7.66 – 8.06 (5H, multiplet)

Refernce Example 8

1-(tert-Butyldimethylsilyl)-3-(1-hydroxy-1-methylethyl)-4-phenylthio-2-azetidinone (18.5 g) is dissolved in a mixture of 150 ml of methanol and 20 ml of water, and the mixture is stirred at room temperature. Thereto is added 12 ml of concentrated hydrochloric acid, and the mixture is stirred for 2 hours. The methanol is distilled off under reduced pressure, water is added to the concentrate, and the mixture is extracted with ethyl acetate. The organic layer is washed with a saturated aqueous sodium chloride solution and dried ($Na_2SO_4$). The ethyl acetate solution is stirred with ice cooling, and 18.6 g of m-chloroperbenzoic acid is added thereto, and stirring is continued for an hour. The reaction mixture is washed with an aqueous sodium hydrogen carbonate solution and with a saturated aqueous sodium chrloride solution, and dried ($Na_2SO_4$). The solvent is distilled off under reduced pressure, and the residue is treated with isopropyl ether to give 9.72 g of 3-(1-hydroxy-1-methylethyl)-4-phenylsulfonyl-2-azetidinone as white crystals.

Comparison of infrared absorption spectra and nuclear magnetic resonance spectra reveals identity of this product with that obtained in Reference Example 7.

Reference Example 9

1-(tert-Butyldimethylsilyl)-4-ethylsulfonyl-3-(1-hydroxy-1-methylethyl)-2-azetidinone (1.70 g) is dissolved in a mixture of 45 ml of methanol and 5 ml of water, and the solution is stirred at room temperature. Thereto is added 1.3 ml of concentrated hydrochloric acid, and the reaction is effected for 4 hours. The reaction mixture is neutralized with sodium hydrogen carbonate, and then extracted with chloroform. The organic layer is dried ($MgSO_4$), and the solvent is distilled off under reduced pressure

to give 440 mg of 4-ethylsulfonyl-3-(1-hydroxy-1-methylethyl)-2-azetidinone as an oil.

NMR spectrum (60MHz, in $d_6$-DMSO+$D_2O$): δ 1.14 (3H, singlet), 1.23 (3H, singlet), 1.23 (2H, triplet), 3.12 (2H, quartet), 3.32 (1H, doublet), 4.75 (1H, doublet)

Example 12

To 5 ml of tetrahydrofuran is added 48 mg of sodium hydride (60% in oil), and the mixture is stirred with ice cooling. Thereto is added 230 mg of methyl 2-benzoylpropionate, and the mixture is stirred for 15 minutes and then cooled to -10°C. 3-(1-Hydroxy-1-methylethyl)-4-phenylsulfonyl-2-azetidinone (270 mg) is added, and the mixture is stirred for 20 minutes. The reaction mixture is poured into a saturated aqueous sodium chloride solution, and extracted with ethyl acetate. The organic layer is washed with a saturated aqueous sodium chloride solution and dried ($Na_2SO_4$), and then the solvent is distilled off under reduced pressure. The residue is passed through a silica gel column. Elution with a 1:1 mixture of chloroform and ethyl acetate gives 240 mg of 4-(1-benzoyl-1-methoxycarbonylethyl)-3-(1-hydroxy-1-methylethyl)-2-azetidinone as an oil.

Infrared absorption spectrum (KBr): 1740 $cm^{-1}$

NMR spectrum (60MHz, in $CDCl_3$): δ 1.25 (3H, singlet), 1.34 (3H, singlet), 1.60 (3H, singlet), 3.09 (1H, doublet), 3.64 (3H, singlet), 4.34 (1H, doublet), 6.65 (1H, broad singlet 7.30 - 7.90 (5H, multiplet)

Example 13

Methyl 2-benzoylpropionate (211 mg) is dissolved in 10 ml of tetrahydrofuran, and the solution is cooled to -10°C and stirred. Thereto is added 44 mg of sodium hydride (60% in oil). The mixture is stirred for 5 minutes, to which is added a solution of 221 mg of 4-ethylsulfonyl-3-(1-hydroxy-1-methylethyl)-2-azetidinone in 4 ml of tetrahydrofuran. After stirring for 30 minutes, the reaction mixture is poured into a saturated aqueous sodium chloride solution, and extracted with ethyl acetate. The organic layer is washed with water and dried ($MgSO_4$), and the solvent is distilled off under reduced pressure. The residue is allowed to pass through a silica gel column. Elution with a 1:1 mixture of chloroform and ethyl acetate gives 96 mg of 4-(1-benzoyl-1-methoxycarbonylethyl)-3-(1-hydroxy-1-methylethyl)-2-azetidinone as an oil.

Comparison of infrared absorption and nuclear magnetic resonance spectra reveals identity of this product with that obtained in Example 12.

Example 14

3-(1-Hydroxy-1-methylethyl)-4-phenylsulfonyl-2-azetidinone (2.70 g) and 3.42 g of 2,4-dioxo-3-methylpentane are dissolved in 50 ml of tetrahydrofuran, and the solution is cooled to -5°C and stirred. Thereto is added dropwise over 30 minutes 10 ml of 1N sodium hydroxide. After allowing the reaction to proceed at the same temperature for an hour, the reaction mixture is poured into a saturated aqueous sodium chloride solution, and extracted with ethyl acetate. The organic layer is washed with a saturated aqueous sodium chloride solution and dried ($Na_2SO_4$), and the solvent is distilled off under reduced pressure. The residue is allowed to pass through a silica gel column. Elution with ethyl acetate gives 1.17 g of 4-(1,1-diacetylethyl)-3-(1-hydroxy-1-methylethyl)-2-azetidinone as an oil. This is crystallized from ether.

Melting point: 115-116°C

Elemental analysis

Calculated for $C_{12}H_{19}NO_4$: C 59.73; H 7.94; N 5.81

Found: C 59.75; H 7.98; H 5.58

Infrared absorption spectrum (KBr): 1735cm$^{-1}$, 1720cm$^{-1}$, 1695cm$^{-1}$

NMR spectrum (60 MHz, in $CDCl_3$): δ 1.26 (3H, singlet), 1.34 (3H, singlet), 1.42 (3H, singlet), 2.18 (3H, singlet), 2.24 (3H, singlet), 2.94 (1H, doublet), 4.24 (1H, doublet), 6.70 (1H, broad singlet)

Example 15

3-Methyl-2,4-dioxopentane (100 mg) is dissolved in 10 ml of tetrahydrofuran, and the solution is cooled to -10°C. Thereto is added 35 mg of sodium hydride (60% in oil), and the mixture is stirred for 10 minutes. Thereto is added a solution of 200 mg of 3-tritylamino-4-methylsulfonyl-2-azetidinone in 2 ml of tetrahydrofuran. After stirring for 40 minutes, the reaction mixture is poured into water, and extracted with ethyl acetate. The organic layer is washed with water and dried ($MgSO_4$), and the solvent is distilled off under reduced pressure. The residue is subjected to thin layer chromatography [developing solvent: ethyl acetate-n-hexane (1:1)], giving 75 mg of 3-tritylamino-4-(1,1-diacetylethyl)-2-azetidinone as a white powder.

Infrared absorption spectrum (KBr): 1790cm$^{-1}$, 1710cm$^{-1}$, 1755cm$^{-1}$

NMR spectrum (60 MHz, in $CDCl_3$): δ 0.96 (3H, singlet), 1.98 (6H, singlet), 3.69 (1H, doublet), 4.15 (1H, doublet), 7.00~7.46 (15H, multiplet)

Example 16

(1) 4-(1,1-Diacetylethyl)-2-azetidinone (0.86 g) and 1.55 g of p-nitrobenzyl glyoxylate monohydrate are dissolved in 20 ml of tetrahydrofuran. To the solution are added 11 g of molecular sieves (3 Å), and the mixture is allowed to stand at room temperature for 8 days. The molecular sieves are filtered off, and the filtrate is concentrated under reduced pressure. The residue is allowed to pass through a silica gel column. Elution with a 2:1 mixture of ethyl acetate and n-hexane gives 0.96 g of 4-(1,1-diacetylethyl)-1-(1-p-nitrobenzyloxycarbonyl-1-hydroxymethyl)-2-azetidinone as an oil.

(2) The 4-(1,1-diacetylethyl)-1-(1-p-nitrobenzyloxycarbonyl-1-hydroxymethyl)-2-azetidinone (5.986 g) prepared by the method of (1) above is dissolved in 100 ml of tetrahydrofuran, and the solution is cooled to -5°C to -10°C in a nitrogen atmosphere and stirred. Thereto are added 3.6 g of 2,6-lutidine, and then dropwise 3.64 g of thionyl chloride. After the addition, the reaction is allowed to proceed at the same temperature for 10 minutes, and then 100 ml of benzene is added to the reaction mixture. The precipitate is filtered off, and the filtrate is concentrated to dryness under reduced pressure to give a crude product of 4-(1,1-diacetylethyl)-1-(1-p-nitrobenzyloxycarbonyl-1-chloromethyl)-2-azetidinone. This is, without purification, dissolved in 180 ml of dried dioxane. Thereto are added 3.27 g of 2,6-lutidine and 8 g of triphenylphosphine, and the mixture is stirred at 50°C in a nitrogen atmosphere for 23 hours. The insoluble matter is filtered off, and the filtrate is concentrated under reduced pressure. The residue is allowed to pass through a silica-gel column. Elution with a 1:1 mixture of ethyl acetate and n-hexane gives 4-(1,1-diacetylethyl)-1-(1-p-nitrobenzyloxycarbonyl-1-triphenylphosphoranylidenemethyl)-2-azetidinone as a powder. This is dissolved in 200 ml of toluene, 20 mg of hydroquinone is added, and the reaction is effected at 90°C in a nitrogen atmosphere for 4 hours. The solvent is distilled off under reduced pressure, and the residue is allowed to pass through a silica gel column. Elution with a 1:1 mixture of chloroform and ethyl acetate gives an oil. This is crystallized from a mixture of chloroform and ether to give 1.10 g of p-nitrobenzyl 1-acetyl-1,2-dimethylcarba-2-penem-3-carboxylate as crystals.

Melting point: 144-145°C

Elemental analysis

Calculated for $C_{18}H_{18}N_2O_6$: C 60.33; H 5.06; N 7.82

Found: C 60.34; H 5.13; N 7.83

Infrared absorption spectrum (KBr): 1700cm$^{-1}$. 1720cm$^{-1}$, 1780cm$^{-1}$

NMR spectrum (90MHz, in $CDCl_3$): : δ 1.30 (3H, singlet), 2.03 (3H, singlet), 2.23 (3H, singlet), 3.20 (2H, doublet of AB quartet), 4.26 (1H, doublet of doublet), 5.35 (2H, quartet), 7.64 (2H, doublet), 8.21 (2H, doublet)

Example 17

p-Nitrobenzyl 1-acetyl-1,2-dimethylcarba-2-penem-3-carboxylate (0.36 g) is dissolved in 30 ml of ethyl acetate. To the solution are added a solution of 0.126 g of sodium hydrogen carbonate in 15 ml of water and 0.36 g of 10% palladium-carbon, and hydrogen is allowed to pass through the mixture at room temperature so as to effect catalytic reduction. After 45 minutes of reaction, the catalyst is filtered off, and the filtrate is allowed to separate into

layers. The aqueous layer is allowed to pass through an Amberlite XAD-2 column. Elution with water is followed by lyophilization. There is obtained 0.10 g of sodium 1-acetyl-1,2-dimethylcarba-2-penem-3-carboxylate as a colorless powder.

Elemental analysis

Calculated for $C_{11}H_{12}NO_4Na \cdot 1.5H_2O$: C 48.53; H 5.55; N 5.15

Found: C 48.10; H 5.51; N 5.07

Infrared absorption spectrum (KBr): $1775cm^{-1}$, $1740cm^{-1}$, $1703cm^{-1}$

NMR spectrum (90MHz, in $D_2O$): : δ 1.39 (3H, singlet), 1.98 (3H, singlet), 2.44 (3H, singlet), 3.35 (2H, doublet of AB quartet), 4.53 (1H, doublet of doublet)

Example 18

(1) 4-(1,-Diacetylpropyl)-2-azetidinone (3.8 g) and 5.3 g of p-nitrobenzyl glyoxylate monohydrate are dissolved in 100 ml of benzene, and the solution is refluxed using a Dean-Stark apparatus for 7 hours. Then, the solvent is distilled off under reduced pressure, and the residue is allowed to pass through a silica-gel column. Elution with a 4:1 mixture of dichloromethane and ethyl acetate gives 7.70 g of 4-(1,1-diacetylpropyl)-1-(1-p-nitrobenzyloxycarbonyl-1-hydroxymethyl)-2-azetidinone as a powder.

(2) 4-(1,1-Diacetylpropyl)-1-(1-p-nitrobenzyloxycarbonyl-1-hydroxymethyl)-2-azetidinone (7.70 g) is dissolved in 100 ml of tetrahydrofuran, and the solution is cooled to -5°C to -10°C in a nitrogen atmosphere and stirred. Thereto are added 6.72 g of 2,6-lutidine and then 6.78 g of thionyl chloride. After 10 minutes of reaction, 100 ml of benzene is added to the reaction

mixture, the precipitate is filtered off, and the filtrate is concentrated under reduced pressure to give a crude product of 4-(1,1-diacetylpropyl)-1-(1-p-nitrobenzyloxycarbonyl-1-chloromethyl)-2-azetidinone as a powder.

This powder, without purification, is dissolved in 100 ml of dioxane, then 4.07 g of 2,6-lutidine and 10 g of triphenylphosphine are added, and the mixture is stirred at 50°C in a nitrogen atmosphere for 18 hours. The insoluble matter is filtered off, and the filtrate is concentrated under reduced pressure. The residue is allowed to pass through a silica gel column. Elution with chloroform gives 4-(1,1-diacetylpropyl)-1-(1-p-nitrobenzyloxycarbonyl-1-triphenylphosphoranylidenemethyl)-2-azetidinone as a powder.

This powder is dissolved in 150 ml of toluene, 20 mg of hydroquinone is added, and the reaction is conducted at 90°C in a nitrogen atmosphere for 18 hours. The reaction mixture is concentrated under reduced pressure, and the residue is allowed to pass through a silica gel column. Elution with a 1:1 mixture of chloroform and ethyl acetate gives an oil, which is crystallized from ether. There is thus obtained 435 mg of p-nitrobenzyl 1-acetyl-1-ethyl-2-methylcarba-2-penem-3-carboxylate.

Melting point: 148-150°C

Elemental analysis

Calculated for $C_{19}H_{20}N_2O_6$: C 61.28; H 5.41; N 7.52

Found: C 60.94; H 5.36; N 7.45

Infrared absorption spectrum (KBγ): 1690cm$^{-1}$, 1722cm$^{-1}$, 1780cm$^{-1}$

NMR spectrum (90 MHz, in $CDCl_3$): $\delta$ 0.86 (3H, triplet), 1.50 - 2.15 (2H, multiplet), 2.03 (3H, singlet), 2.22 (3H, singlet), 3.30 (2H, doublet of AB quartet), 4.42 (1H, doublet

of doublet ), 5.35 (2H, AB quartet ), 7.64 (2H, doublet ), 8.22 (2H, doublet )

Example 19

4-(1-Acetyl-1-ethoxycarbonylethyl)-2-azetidinone (4.26 g) and 4.99 g of p-nitrobenzyl glyoxylate monohydrate are dissolved in 150 ml of benzene, and the solution is refluxed using a Dean-Stark apparatus fpr 20 hours. The solvent is distilled off under reduced pressure, and the residual oil is purified by allowing it to pass through a silica gel column. Elution with a 4:1 mixture of dichloromethane and ethyl acetate gives 8.211 g of 4-(1-acetyl-1-ethoxycarbonylethyl)-1-(1-p-nitrobenzyloxycarbonyl-1-hydroxymethyl)-2-azetidinone as an oil. This is dissolved in 100 ml of tetrahydrofuran, 6.73 ml of 2,6-lutidine is added, and the mixture is cooled to -10°C and stirred. Then, thereto is added 2.82 ml of thionyl chloride dropwise. The reaction is then conducted for 10 minutes, and the reaction mixture is poured into water and extracted with ethyl acetate. The organic layer is washed with water and dried ($MgSO_4$), and the solvent is distilled off under reduced pressure to give 7.48 g of 4-(1-acetyl-1-ethoxycarbonylethyl)-1-(1-p-nitrobenzyloxycarbonyl-1-chloromethyl)-2-azetidinone as an oil. This oil is dissolved in 70 ml of N,N-dimethylformamide. To the solution are 8.90 g of triphenylphosphine, 2.55 g of sodium iodide and 2.2 ml of 2,6-lutidine, and the mixture is stirred at room temperature in a nitrogen atmosphere for 18 hours. The reaction mixture is poured into water and extracted with ethyl acetate. The organic layer is washed with water and dried ($MgSO_4$), and the solvent is distilled off under reduced pressure. The residue is purified by allowing it to pass through a silica-gel column. Elution with chloroform

gives 9.00 g of 4-(1-acetyl-1-ethoxycarbonylethyl)-1-(1-p-nitrobenzyloxycarbonyl-1-triphenylphosphoranylidenemethyl)-2-azetidinone as a powder. 5.00 g of this product is dissolved in 350 ml of toluene and the reaction is conducted at 90°C in a nitrogen atmosphere for 17 hours. The solvent is distilled off under reduced pressure, and the residue is purified by allowing to through a silica-gel column. Elution with chloroform gives 2.14 g of a 1: 1 mixture of isomers of p-nitrobenzyl 1-ethoxycarbo 1,2-dimethylcarba-2-penem-3-carboxylate as an oil.

NMR spectrum (90 MHz, in $CDCl_3$): : δ 1.26 (3H × $\frac{1}{2}$; triplet), 1.27 (3H × $\frac{1}{2}$, triplet), 1.36 (3H × $\frac{1}{2}$, singlet), 1.49 (3H × $\frac{1}{2}$, singlet), 2.06 (3H × $\frac{1}{2}$, singlet), 2.10 (3H × $\frac{1}{2}$, singlet), 3.10 (2H × $\frac{1}{2}$, doublet of AB quartet), 3.17 (2H × $\frac{1}{2}$, doublet of AB quartet), 3.86 (1H × $\frac{1}{2}$, doublet of doublet), 4.20 (2H × $\frac{1}{2}$, AB quartet), 4.22 (2H × $\frac{1}{2}$, AB quartet), 4.47 (1H × $\frac{1}{2}$, doublet of doublet), 5.37 (2H, AB quartet). 7.66 (2H, doublet), 8.25 (2H, doublet)

Crystallization of this 1:1 isomeric mixture from ether gives one of the isomers as colorless crystals.

Melting point: 120-122°C

Elemental analysis

Calculated for $C_{19}H_{20}N_2O_7$: C 58.76; H 5.19; N 7.21

Found: C 58.90; H 5.10; N 7.20

Infrared absorption spectrum (KBr): 1780cm$^{-1}$, 1720cm$^{-1}$

NMR spectrum (90MHz, in $CDCl_3$) : δ 1.26 (3H, .

triplet ), 1.49 (3H, singlet ), 2.06 (3H, singlet ), 3.10 (2H, doublet of AB quartet ), 3.86 (1H, doublet of doublet ), 4.20 (2H, AB quartet ), 5.37 (2H, AB quartet ), 7.66 (2H, doublet ), 8.25 (2H, doublet )

Example 20

(1) 4-(1-Benzoyl-1-methoxycarbonylethyl)-2-azetidinone (5.33 g) and 5.77 g of p-nitrobenzyl glyoxylate monohydrate are dissolved in 180 ml of benzene, and the solution is refluxed using a Dean-Stark apparatus for 8 hours. The solvent is distilled off under reduced pressure, and the residue is allowed to pass through a silica-gel column. Elution with a 2:1 mixture of dichloromethane and ethyl acetate gives 8.00 g of 4-(1-benzoyl-1-methoxycarbonylethyl)-1-(1-p-nitrobenzyloxycarbonyl-1-hydroxymethyl)-2-azetidinone as an oil.

This oil is dissolved in 80 ml of tetrahydrofuran, and the solution is cooled to -5° to -10° in a nitrogen atmosphere and stirred. To the solution is added 4.33 ml of 2,6-lutidine, to which is added dropwise 2.47 ml of thionyl chloride followed by allowing the reaction to proceed at the same temperature for 20 minutes. The reaction mixture is then poured into water and extracted with ethyl acetate. The organic layer is washed with a 5% aqueous solution of sodium hydrogen carbonate and with a saturated aqueous solution of sodium chloride, and dried ($Na_2SO_4$). The solvent is distilled off under reduced pressure. There is thus obtained 7.3 g of 4-(1-benzoyl-1-methoxycarbonylethyl)-1-(1-p-nitrobenzyloxycarbonyl-1-chloromethyl)-2-azetidinone as an oil. 4.89 g of this oil is dissolved in 40 ml of N,N-dimethylformamide. To the solution are added 1.15 ml of 2,6-lutidine, 5.25 g of triphenylphosphine and 1.5 g of sodium iodide in this order, and the mixture is stirred at room temperature in a nitrogen atmosphere for 17 hours. The reaction mixture is poured into water and extracted with ethyl acetate. The organic layer is washed with a saturated aqueous solution of sodium chloride, and dried ($Na_2SO_4$). The solvent is distilled off under reduced pressure, and the residue is allowed to pass through a silica gel column. Elution with a 1: 1 mixture of chloroform and ethyl acetate gives 4-(1-benzoyl-1-methoxycarbonylethyl)-1-(1-p-nitrobenzyloxycarbonyl-1-triphenylphosphoranylidene-methyl)-2-azetidinone as a powder. This is dissolved in 150 ml of toluene, 20 mg of hydroquinone is added, and the reaction is conducted at 90°C in a nitrogen atmosphere for 20 hours. The solvent is distilled off under reduced pressure, and the residue is allowed to pass through a silica gel column. Elution with a 6:1 mixture of chloroform and ethyl acetate gives an oil. This is crystallized from a mixture of dichloromethane and ether to give 2.30 g of a 7:3 mixture of isomers of p-nitrobenzyl 1-methyl-1-methoxycarbonyl-2-phenylcarba-2-penem-3-carboxylate.

NMR spectrum (60MHz, in $CDCl_3$) : $\delta$ 1.38 (3H × $\frac{3}{10}$, singlet), 1.46 (3H × $\frac{7}{10}$, singlet), 2.65 – 3.59 (2H, multiplet), 3.65 (3H × $\frac{7}{10}$, singlet), 3.83 (3H × $\frac{3}{10}$, singlet), 4.13 (1H × $\frac{3}{10}$, doublet of doublet), 4.74 (1H × $\frac{7}{10}$, doublet of doublet), 5.14 (2H, AB quartet), 6.90 – 7.35 (7H, multiplet), 8.01 (2H, doublet)

Recrystallization of this product from ether gives one of the isomers as colorless crystals.

Melting point: 115-121°C

Elemental analysis

Calculated for $C_{23}H_{20}N_2O_7$: C 63.30; H 4.62; N 6.42

Found: C 63.39; H 4.47; N 6.29

Infrared absorption spectrum (KBr): $1720cm^{-1}$, $1735cm^{-1}$, $1790cm^{-1}$

NMR spectrum (90MHz, in $CDCl_3$): δ 1.42 (3H, singlet), 3.26 (2H, doublet of AB quartet), 3.61 (3H, singlet), 4.71 (1H, doublet of doublet), 5.14 (2H, AB quartet), 7.00～7.35 (7H, multiplet), 8.07 (2H, doublet)

Example 21

p-Nitrobenzyl 1-methyl-1-methoxycarbonyl-2-phenylcarba-2-penem-3-carboxylate (7:3 isomeric mixture, 437 mg) is dissolved in 30 ml of ethyl acetate. To the solution are added a solution 0.10 g of sodium hydrogen carbonate in 15 ml of water and 440 mg of 10% palladium-carbon, and catalytic reduction is conducted at room temperature under atmospheric pressure for an hour and 45 minutes. The catalyst is filtered off, the filtrate is allowed to separate into layers, and the aqueous layer is taken. The aqueous layer is lyophilized, and the residue is dissolved in water and is allowed to pass through a Sephadex LH-20 (Rohm & Haas Co.) column Elution with water is followed by lyophilization of fractions containing the objective compound. There is obtained 275 mg of a 7:3 mixture of isomers of sodium 1-methyl-1-methoxycarbonyl-2-phenyl-carba-2-penem-3-carboxylate as a colorless powder.

Infrared absorption spectrum (KBr): 1725$cm^{-1}$, 1760$cm^{-1}$

NMR spectrum (90 MHz, in $d_6$-DMSO : δ 1.24 (3H × $\frac{3}{10}$, singlet ), 1.30 (3H × $\frac{7}{10}$, singlet ), 3.14 (2H, multiplet ), 3.54 (3H × $\frac{7}{10}$, singlet ), 3.73 (3H × $\frac{3}{10}$, singlet ), 3.90 (1H × $\frac{3}{10}$, doublet of doublet ), 4.44 (1H × $\frac{7}{10}$, doublet of doublet ), 7.17 (5H, singlet )

Example 22

4-(1-Benzoyl-1,1-diethoxycarbonylmethyl)-2-azetidinone (1.33 g) and 1.09 g of p-nitrobenzyl glyoxylate monohydrate are dissolved in 50 ml of benzene, and the solution is refluxed using a Dean-Stark apparatus for 8 hours. The solvent is distilled off under reduced pressure, and the residue is allowed to pass through a silica gel column. Elution with a 6:1 mixture of dichloromethane and ethyl acetate gives 0.869 g of 4-(1-benzoyl-1,1-diethoxycarbonylmethyl)-1-(1-p-nitrobenzyloxycarbonyl-1-hydroxymethyl)-2-azetidinone as an oil.

This oil is dissolved in 12 ml of tetrahydrofuran, and the solution is cooled to -10°C to -5°C in a nitrogen atmosphere and stirred. To the solution is added 0.45 ml of 2,6-lutidine, and then 0.232 ml of thionyl chloride is added dropwise. The reaction is then allowed to proceed for 10 minutes, and the reaction mixture is poured into water and extracted with ethyl acetate. The organic layer is washed in sequence with water, a 5% aqueous solution of sodium hydrogen carbonate and a saturated aqueous solution of sodium chloride, and dried ($Na_2SO_4$). Removal of the solvent by distillation under reduced pressure gives 4-(1-benzoyl-1,1-diethoxycarbonylmethyl)-1-(1-p-nitrobenzyloxycarbonyl-1-chloromethyl)-2-azetidinone as an oil.

This oil is dissolved in 12 ml of N,N-dimethylformamide. Thereto are added in sequence 0.22 ml of 2,6-lutidine, 0.84 g of triphenylphosphine and 0.24 g of sodium iodide, and the mixture is stirred at room temperature in a nitrogen atmosphere for 21 hours. The reaction mixture is poured into water and extracted with ethyl acetate. The organic layer is washed with water and a saturated aqueous solution of sodium chloride, and dried ($Na_2SO_4$). The solvent is distilled off under reduced pressure, and the residue is allowed to pass through a silica-gel column. Elution with a 6:1 mixture of chloroform and ethyl acetate gives 4-(1-benzoyl-1,1-diethoxycarbonylmethyl)-1-(1-p-nitrobenzyloxycarbonyl-1-triphenylphosphoranylidenemethyl)-2-azetidinone as a powder. This is dissolved in 50 ml of toluene, 20 mg of hydroquinone is added, and the reaction is conducted at 90°C in a nitrogen atmosphere for 15.5 hours. The solvent is then distilled off under reduced pressure, and the residue is allowed to pass through a silica gel column. Elution with a 9:1 mixture of chloroform and ethyl acetate gives an oil. This is crystallized from isopropyl ether to give 119 mg of p-nitrobenzyl 1,1-diethoxycarbonyl-2-phenylcarba-2-penem-3-carboxylate.

Melting point: 108-111°C

Elemental analysis

Calculated for $C_{26}H_{24}N_2O_9$: C 61.41; H 4.76; n 5.51

Found: C 61.45; H 4.64; N 5.59

Infrared absorption spectrum (KBr): 1725cm$^{-1}$, 1795cm$^{-1}$

NMR spectrum (90MHz, in $CDCl_3$): $\delta$ 0.83 (3H,

triplet ), 1.30 (3H, triplet ), 2.90 (1H doublet of doublet ), 3.55 (1H, doublet of doublet ), 3.94 (2H multiplet ), 4.34 (2H quartet ), 5.17 (3H, AB quartet and doublet of doublet ), 7.05 – 7.35 (7H, multiplet ), 8.08 (2H, doublet )

Example 23

4-(1-Ethylthiocarbonyl-1-methoxycarbonylethyl)-2-azetidinone (2.45 g) and 2.72 g of p-nitrobenzyl glyoxylate monohydrate are dissolved in 80 ml of benzene, and the solution is refluxed using a Dean-Stark apparatus for 9 hours. The solvent is distilled off under reduced pressure, and the residue is passed through a silica-gel column. Elution with a 8:2 mixture of dichloromethane and ethyl acetate gives 3.695 g of 4-(1-ethylthiocarbonyl-1-methoxycarbonylethyl)-1-(1-p-nitrobenzyloxycarbonyl-1-hydroxymethyl)-2-azetidinone as an oil. This is dissolved in 60 ml of tetrahydrofuran, and the solution is cooled to -10°C in a nitrogen atmosphere and stirred. Thereto is added 2.04 ml of 2,6-lutidine, and then 1.16 ml of thionyl chloride is added dropwise. After the addition, the reaction is conducted for 10 minutes. The reaction mixture is poured into water and extracted with ethyl acetate. The organic layer is washed with water, a 5% aqueous solution of sodium hydrogen carbonate and a saturated aqueous solution of sodium chloride, and then dried ($MgSO_4$). The solvent is distilled off under reduced pressure to give 4-(1-ethylthiocarbonyl-1-methoxycarbonylethyl)-1-(1-p-nitrobenzyloxycarbonyl-1-chloromethyl)-2-azetidinone as an oil. This oil is dissolved in 50 ml of N,N-dimethylformamide. To the solution are added 0.925 ml of 2,6-lutidine, 4.192g of triphenylphosphine and 1.2g of sodium iodide in this

order. The mixture is stirred at room temperature in a nitrogen atmosphere for 17 hours. The reaction mixture is poured into water and extracted with ethyl acetate. The organic layer is washed with water and with a saturated aqueous solution of sodium chloride, and dried ($MgSO_4$). The solvent is distilled off under reduced pressure, and the residue is allowed to pass through a silica gel column. Elution with a 1:1 mixture of n-hexane and ethyl acetate gives 1.162 g of 4-(1-ethylthiocarbonyl-1-methoxycarbonylethyl)-1-(1-p-nitrobenzyloxycarbonyl-1-triphenylphosphoranylidenemethyl)-2-azetidinone as a powder. This powder is dissolved in 100 ml of xylene, 20 mg of hydroquinone is added, and the mixture is stirred at 140°C in a nitrogen atmosphere for 22 hours. The reaction mixture is concentrated under reduced pressure, and the residue is allowed to pass through a silica gel column. Elution with a 4:1 mixture of chloroform and ethyl acetate gives an oil. This oil is crystallized from ether to give 190 mg of p-nitrobenzyl 1-methyl-1-methoxycarbonyl-2-ethylthiocarba-2-penem-3-carboxylate.

Melting point: 131-132°C

Elemental analysis

Calculated for $C_{19}H_{20}N_2O_7S$: C 54.28; H 4.79; N 6.66

Found: C 54.33; H 4.63; N 6.67

Infrared absorption spectrum (KBr): 1730cm $^{-1}$, 1790cm$^{-1}$

NMR spectrum (90MHz, in $CDCl_3$): δ 1.20 (3H, triplet), 1.46 (3H, singlet), 2.87 (2H, AB quartet), 3.23 (2H, doublet of AB quartet), 3.77 (3H singlet), 4.37 (1H, doublet of doublet), 5.37 (2H, AB quartet), 7.64 (2H, doublet), 8.24 (2H, doublet)

Example 24

p-Nitrobenzyl 1-methyl-1-methoxycarbonyl-2-ethylthiocarba-2-penem-3-carboxylate (0.21 g) is dissolved in 15 ml of ethyl acetate. To the solution are added a solution of 51 mg of sodium hydrogen carbonate in 8 ml of water and 0.25 g of 10% palladium-carbon, and catalytic reduction is conducted at room temperature for an hour. The catalyst is then filtered off, and the filtrate is allowed to separate into two layers. The aqueous layer is taken and allowed to pass through a Sephadex LH-20 column. Elution with water and lyophilization of the fractions containing the objective compound give 44 mg of sodium 1-methyl-1-methoxycarbonyl-2-ethylthiocarba-2-penem-3-carboxylate as a powder.
Infrared absorption spectrum (KBr): $1730cm^{-1}$, $1760cm^{-1}$

Example 25

p-Nitrobenzyl 1-acetyl-1-ethyl-2-methylcarba-2-penem-3-carboxylate (200 mg) is dissolved in 20 ml of ethyl acetate. To the solution are added 200 mg of 10% palladium-carbon and a solution of 54 mg of sodium hydrogen carbonate in 10 ml of water, and catalytic reduction is carried out at room temperature and at normal pressure for an hour. The catalyst is filtered off, and the filtrate is separated into two layers. The aqueous layer is taken and lyophilized, and the residue is allowed to pass through a Sephadex LH-20 column. Elution with water and lyophilization of the fractions containing the objective compound give 114 mg of sodium 1-acetyl-1-ethyl-2-methylcarba-2-penem-3-carboxylate as a powder.

Infrared absorption spectrum (KBr): $1750cm^{-1}$, $1703cm^{-1}$

NMR spectrum (60MHz,in $D_2O$) : δ 0.82 (3H, triplet), 1.65 – 2.10 (2H, multiplet), 1.84 (3H, singlet), 2.32 (3H, singlet), 3.36 (2H, doublet of AB quartet), 4.48 (1H, doublet of doublet)

Example 25

p-Nitrobenzyl 1-ethoxycarbonyl-1,2-dimethylcarba-2-penem-3-carboxylate (388 mg) is dissolved in 20 ml of ethyl acetate. To the solution are added 400 mg of 10% palladium-carbon and a solution of 100 mg of sodium hydrogen carbonate in 10 ml of water, and catalytic reduction is conducted at room temperature and at normal pressure for an hour. The catalyst is filtered off, the filtrate is allowed to separate into two layers, and the aqueous layer is taken and lyophilized. The freeze-dried residue is dissolved in water and the solution is allowed to pass through a Sephadex LH 20 column. Elution with water and lyophilization of the fractions containing the objective compound give 180 mg of sodium 1-ethoxycarbonyl-1,2-dimethylcarba-2-penem-3-carboxylate (1:1 mixture of diastereomers) as a powder.

Infrared absorption spectrum (KBr): $1755cm^{-1}$, $1725cm^{-1}$

NMR spectrum (60MHz, in $D_2O$) : δ 1.00 – 1.35 (3H, mutiplet), 1.30 (3H×½, singlet), 1.46 (3H, singlet), 2.79 – 3.65 (2H, multiplet), 3.99 (1H×½, doublet of doublet), 4.24 (2H, AB quartet), 4.50 (1H×½, doublet of doublet)

Example 27

p-Nitrobenzyl 1,1,-diethoxycarbonyl-2-phenylcarba-2-penem-3-carboxylate (300 mg) is dissolved in 30 ml of ethyl acetate. To the solution are added 0.30 g of 10% palladium-carbon and a solution of 55 mg of sodium hydrogen carbonate in 15 ml of water, and catalytic reduction is conducted at room temperature at normal pressure for an hour. The catalyst is filtered off, the filtrate is allowed to separate into two layers, and the aqueous layer is taken and lyophilized. The residue is dissolved in water and the solution is allowed to pass through a Sephadex LH-20 column. Elution with water is followed by lyophilization of the fractions containing the objective compound. There is obtained 140 mg of sodium 1,1-diethoxycarbonyl-2-phenylcarba-2-penem-3-carboxylate as a powder.

Infrared absorption spectrum (KBr): 1770$cm^{-1}$, 1715$cm^{-1}$

NMR spectrum (90MHz, in $D_2O$) . δ 0.77 (3H, triplet), 1.22 (3H, triplet), 2.74 (1H, doublet of doublet), 3.49 (1H, doublet of doublet), 3.86 (2H doublet of AB quartet), 4.39 (2H, doublet of AB quartet), 4.87 (1H, doublet of doublet), 7.23 (5H, singlet)

Example 28

4-(1-Benzoyl-1-methoxycarbonylethyl)-3-(1-hydroxyl-methylethyl)-2-azetidinone (1.39 g) and 1.49 g of p-nitrobenzyl glyoxylate monohydrate are dissolved in 50 ml of benzene, and the solution is refluxed using a Dean-Stark apparatus for 17 hours. The reaction mixture is concentrated under reduced pressure, and the residue is allowed to pass through a silica-gel column. Elution with a 5:1 mixture of dichloromethane and acetone gives

1.60 g of 4-(1-benzoyl-1-methoxycarbonylethyl)-3-(1-hydroxy-1-methylethyl)-1-(1-p-nitrobenzyloxycarbonyl-1-hydroxymethyl)-2-azetidinone as a powder. This product is a mixture of four isomers. Crystallization of this product from ether gives 300 mg of crystals, which presumably consist of a mixture of two isomers (a 5:6 mixture according to the nuclear magnetic resonance spectrum).

Melting point: 141-143°C

Elemental analysis

Calculated for $C_{26}H_{28}N_2O_{10}$: C 59.08; H 5.40; N 5.30

Found: C 58.81; H 5.32; N 5.13

Infrared absorption spectrum (KBr): $1760cm^{-1}$, $1740cm^{-1}$, $1680cm^{-1}$

NMR spectrum (90MHz, $CDCl_3$+in $D_2O$): δ 1.06 (3H × $\frac{5}{11}$, singlet), 1.20 (3H, singlet), 1.25 (3H × $\frac{6}{11}$, singlet), 1.53 (3H × $\frac{6}{11}$, singlet), 1.60 (3H × $\frac{5}{11}$, singlet), 3.03 (1H × $\frac{6}{11}$, doublet), 3.06 (1H × $\frac{5}{11}$, doublet), 3.60 (3H, singlet), 4.64 (1H × $\frac{6}{11}$, doublet), 4.68 (1H × $\frac{5}{11}$, doublet), 5.21 (2H × $\frac{6}{11}$, singlet), 5.28 (2H × $\frac{5}{11}$, singlet), 5.40 (1H × $\frac{5}{11}$, singlet), 5.68 (1H × $\frac{6}{11}$, singlet), 7.30 – 8.25 (9H, multiplet)

4-(1-Benzoyl-1-methoxycarbonylethyl)-3-(1-hydroxy-1-methylethyl)-1-(1-p-nitrobenzyloxycarbonyl-1-hydroxymethyl)-2-azetidinone (mixture of four isomers) (0.276 g) is dissolved in 10 ml of tetrahydrofuran. The solution is cooled to -15°C in a nitrogen atmosphere and stirred. To the solution are added 0.09 ml of 2,6-lutidine and then 0.047 ml of thionyl chloride. After

stirring for 10 minutes, dichloromethane is added to the reaction mixture, and the resulting solution is washed with water and a saturated aqueous solution of sodium chloride, and dried ($Na_2SO_4$). The solvent is distilled off under reduced pressure. There is obtained 4-(1-benzoyl-1-methoxycarbonylethyl)-3-(1-hydroxy-1-methylethyl)-(1-p-nitrobenzyloxycarbonyl-1-chloromethyl)-2-azetidinone as an oil. This oil is dissolved in 5 ml of dimethylformamide. To the solution are added in sequence 0.08 g of sodium iodide, 0.281 g of triphenylphosphine and 0.062 ml of 2,6-lutidine, and the mixture is stirred at room temperature in a nitrogen atmosphere for 16 hours. The reaction mixture is poured into water and extracted with ethyl acetate. The organic layer is washed with water and dried ($Na_2SO_4$). The solvent is distilled off under reduced pressure, and the residue is allowed to pass through a silica gel column. Elution with a 1:9 mixture of ethyl acetate and chloroform and then with ethyl acetate gives 104 mg of 4-(1-benzoyl-1-methoxycarbonylethyl)-3-(1-hydroxy-1-methylethyl)-1-(1-p-nitrobenzyloxycarbonyl-1-triphenylphosphoranylidenemethyl)-2-azetidinone. This is dissolved in 5 ml of toluene. To the solution is added 3 mg of hydroquinone, and the mixture is stirred at 90°C in a nitrogen atmosphere for 18 hours. The solvent is distilled off, and the residue is allowed to pass through a silica-gel column. Elution with a 1:1 mixture of chloroform and ethyl acetate gives 7 mg of p-nitrobenzyl 1-methoxycarbonyl-6-(1-hydroxy-1-methylethyl)-1-methyl-2-phenylcarba-2-penem-3-carboxylate as an oil.

Infrared absorption spectrum (KBr): $1765 cm^{-1}$, $1735 cm^{-1}$, $1725 cm^{-1}$

NMR spectrum (90MHz, in $CDCl_3$): $\delta$ 1.32 (3H,

singlet ), 1.43 (3H, singlet ), 1.48 (3H, singlet ), 3.37 (1H, doublet ), 3.66 (3H, singlet ), 4.65 (1H, doublet ), 5.15 (2H, AB quartet ), 7.00 – 8.20 (9H, multiplet )

Example 29

4-(1,1-Diacetylethyl)-3-(1-hydroxy-1-methylethyl)-2-azetidinone (1.054 g) and 2.00 g of p-nitrobenzyl glyoxylate monohydrate are dissolved in 100 ml of benzene, and the solution is refluxed using a Dean-Stark apparatus for 15 hours. The solvent is distilled off under reduced pressure, and the residue is purified by allowing it to pass through a silica-gel column. Elution with a 2:3 mixture of chloroform and ethyl acetate gives 1.84 g of 4-(1,1-diacetylethyl)-3-(1-hydroxy-1-methylethyl)-1-(1-p-nitrobenzyloxycarbonyl-1-hydroxymethyl)-2-azetidinone as a 1:1 mixture of diastereoisomers. Crystallization from a mixture of chloroform and ether gives one isomer as a pure product. Yield 1.19 g.

Melting point: 168-170°C

Elemental analysis

Calculated for $C_{21}H_{26}N_2O_9 \cdot \frac{1}{3}H_2O$: C 55.25; H 5.89; N 6.14

Found: C 55.19; H 5.67; N 6.23

Infrared absorption spectrum (KBr): 1760cm$^{-1}$, 1735cm$^{-1}$, 1685cm$^{-1}$

NMR spectrum (90MHz, in $d_6$-DMSO): δ 104 (3H, singlet ), 1.06 (3H, singlet ), 1.46 (3H, singlet ), 2.10 (3H, singlet ), 2.20 (3H, singlet ), 2.77 (1H, doublet ), 4.59 (1H, doublet ), 4.97 (1H, doublet ), 5.26 (2H, singlet ), 6.57 (1H, doublet ), 7.76 and 8.26 (4H, doublet of doublet )

4-(1,1-Diacetylethyl)-3-(1-hydroxy-1-methylethyl)-1-(1-p-nitrobenzyloxycarbonyl-1-hydroxymethyl)-2-azetidinone (0.90 g) is dissolved in 50 ml of tetrahydrofuran, and the solution is cooled to -20°C in a nitrogen atmosphere and stirred. To the solution are added in sequence 0.5 ml of lutidine and a solution of 0.3 ml of thionyl chloride in 8 ml of tetrahydrofuran, and the mixture is stirred for 5 minutes. Benzene (100 ml) is added to the reaction mixture, and the insolubles are filtered off. The insoluble matter is washed two 20 ml portions of benzene. The filtrate and the washings are combined and evaporated to dryness under reduced pressure. There is thus obtained 4-(1,1-diacetylethyl)-3-(1-hydroxy-1-methylethyl)-1-(1-p-nitrobenzyloxycarbonyl-1-chloromethyl)-2-azetidinone. This is dissolved in 15 ml of dried dimethylformamide, then 0.34 g of potassium iodide, 1.05 g of triphenylphosphine and 0.5 ml of 2,6-lutidine are added in this order, and the mixture is stirred at 35°C in a nitrogen atmosphere for 16 hours. The reaction mixture is poured into water and extracted with ethyl acetate. The organic layer is washed with water and dried ($Na_2SO_4$). The solvent is distilled off under reduced pressure, and the residue is allowed to pass through a silica-gel column. Elution with a 3:2 mixture of ethyl acetate and n-hexane and then with ethyl acetate gives 420 mg of p-nitrobenzyl 1-acetyl-1,2-dimethyl-6-(1-hydroxyl-methylethyl)carba-2-penem-3-carboxylate as an oil. This is crystallized from ether.

Melting point: 142-144°C

Elemental analysis

| | |
|---|---|
| Calculated for $C_{21}H_{24}N_2O_7$: | C 60.57; H 5.81; N 6.73 |
| Found: | C 60.65; H 5.92; N 6.71 |

Infrared absorption spectrum (KBr): $1780cm^{-1}$, $1710cm^{-1}$

NMR spectrum (90MHz, in $CDCl_3$): δ 1.26 (3H, singlet), 1.33 (3H, singlet), 1.44 (3H, singlet), 1.82 (1H, broad singlet), 2.02 (3H, singlet), 2.36 (3H, singlet), 3.34 (1H, doublet), 4.24 (1H, doublet), 5.40 (2H, AB quartet), 7.70 and 8.26 (4H, doublet of doublet)

Example 30

p-Nitrobenzyl 1-acetyl-1,2-dimethyl-6-(1-hydroxy-1-methylethyl)carba-2-penem-3-carboxylate (200 mg) is dissolved in 20 ml of ethyl acetate. To the solution are added a solution of 48 mg of sodium hydrogen carbonate in 10 ml of water and 200 mg of 10% palladium-carbon, and catalytic reduction is carried out at room temperature at normal pressure for 50 minutes. The catalyst is then filtered off, and the filtrate is allowed to separate into two layers. The aqueous layer is lyophilized, and the powder obtained is dissolved in water and allowed to pass through a Sephadex LH-20 column. Elution with water and lyophilization of the fractions containing the objective compound give 117 mg of sodium 1-acetyl-1,2-dimethyl-6-(1-hydroxy-1-methylethyl)-carba-2-penem-3-carboxylate as a white powder.

Infrared absorption spectrum (KBr). $1740cm^{-1}$, $1695cm^{-1}$

NMR spectrum (60MHz, in $D_2O$). δ 179 (3H, singlet), 1.35 (3H, singlet), 1.41 (3H, singlet), 1.93 (3H, singlet), 240 (3H, singlet), 3.62 (1H, doublet), 434 (1H, doublet)

CLAIMS

1. A carba-2-penem compound having the formula (I):

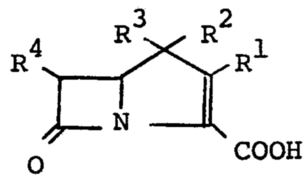

(I),

wherein $R^1$ is an alkyl, aryl, alkylthio, arylthio or acylaminoalkylthio group, $R^2$ and $R^3$ are each independently an alkoxycarbonyl or cyano group or a group of the formula $COR^1$ ($R^1$ being as above defined), $R^3$ may further be an alkyl or aryl group, and $R^4$ is a hydrogen atom, an alkyl, hydroxyalkyl or aryl group or an amino group which may optionally be protected, or a salt or ester thereof.

2. A carba-2-penem compound as claimed in Claim 1, wherein $R^1$ is a $C_{1-6}$ alkyl, phenyl, naphthyl, $C_{1-6}$ alkylthio, phenylthio, naphthylthio or $C_{1-6}$ acylamino-$C_{1-6}$ alkylthio group, $R^2$ and $R^3$ are each independently a $C_{1-6}$ alkoxycarbonyl or cyano group or a group of the formula $COR^1$ ($R^1$ being as above defined), $R^3$ may further be a $C_{1-6}$ alkyl, phenyl or naphthyl group, and $R^4$ is a hydrogen atom, a $C_{1-6}$ alkyl, hydroxy-$C_{1-6}$-alkyl, phenyl or naphthyl or an amino group which may optionally be protected, said $C_{1-6}$ alkyl, phenyl and naphthyl being unsubstituted or substituted with one or more of a $C_{1-2}$ alkoxy-$C_{1-2}$ alkyl, a $C_{1-2}$ alkoxyphenyl, a $C_{1-2}$ alkoxy-naphthyl, a halogeno-$C_{1-2}$ alkyl, a halogenophenyl and a halogenonaphthyl, further said phenyl and naphthyl being unsubstituted or substituted with a $C_{1-6}$ alkyl.

3. A carba-2-penem compound as claimed in Claim 2, wherein a protective group of the protected amino group represented by $R^4$ is a $C_{1-6}$ alkylcarbonyl which may be substituted with a halogen, $C_{1-6}$ alkoxycarbonyl which may be substituted with a halogen, a $C_{1-6}$ alkoxy - $C_{1-6}$ alkylcarbonyl, a $C_{7-19}$ aralkyloxycarbonyl, a $C_{7-19}$ aralkyl or tetrahydropyranyl group.

4. A carba-2-penem compound as claimed in Claim 1, wherein $R^1$ is a $C_{1-4}$ alkyl, phenyl, $C_{1-4}$ alkylthio, phenylthio or a acetylamino-$C_{1-4}$ alkylthio, $R^2$ is a $C_{1-4}$ alkoxycarbonyl, cyano or a $C_{1-4}$ alkylcarbonyl, $R^3$ is a $C_{1-4}$ alkyl, phenyl, a $C_{1-4}$ alkoxycarbonyl or a $C_{1-4}$ alkylcarbonyl, and $R^4$ is a hydrogen atom, $C_{1-4}$ alkyl or phenyl.

5. A carba-2-penem compound as claimed in Claim 1, wherein $R^1$ is a $C_{1-4}$ alkyl, phenyl or a $C_{1-4}$ alkylthio, $R^2$ is a $C_{1-4}$ alkoxycarbonyl, a $C_{1-4}$ alkylcarbonyl or cyano, $R^3$ is a $C_{1-4}$ alkoxycarbonyl, a $C_{1-4}$ alkylcarbonyl, a $C_{1-4}$ alkyl or phenyl, and $R^4$ is a hydrogen atom, a $C_{1-4}$ alkyl or a hydroxyl-$C_{1-4}$ alkyl.

6. A method of producing carba-2-penem compounds of the formula (III):

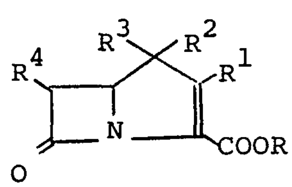

(III),

wherein $R^1$ is an alkyl, aryl, alkylthio, arylthio or acylaminoalkylthio group, $R^2$ and $R^3$ are each independently an alkoxycarbonyl or cyano group or a group of the formula $COR^1$ ($R^1$ being as above defined), $R^3$ may further be an alkyl or aryl group, $R^4$ is a hydrogen atom, an alkyl, hydroxyalkyl or aryl group or an amino group which may optionally be protected, and COOR is a protected carboxyl group,

which comprises subjecting a compound of the formula (II):

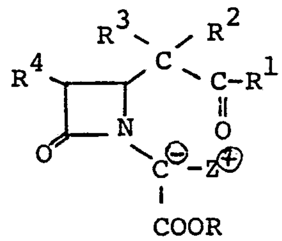

wherein Z is a phosphonio or phosphono group, to ring closure.

7. A method as claimed in Claim 6, for producing a compound (I) as defined in any of claims 1 to 5, wherein said COOR in said compound of formula (III) is optionally converted to COOH by a method known *per se*.

8. A compound having the formula (VI):

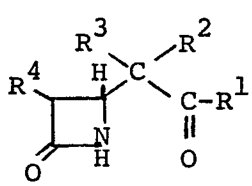

(VI)

wherein $R^1$ is an alkyl, aryl, alkylthio, arylthio or acylaminoalkylthio group, $R^2$ and $R^3$ are each independently an alkoxycarbonyl or cyano group or a group of the formula $COR^1$ ($R^1$ being as above defined), $R^3$ may further be an alkyl or aryl group, and $R^4$ is a hydrogen atom, an alkyl, hydroxyalkyl or aryl group or an amino group which may optionally be protected, or a salt or ester thereof.

9. A method of producing a compound of the formula (VI):

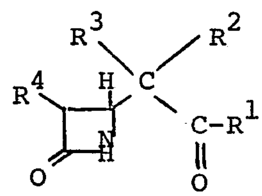

(VI),

wherein $R^1$ is an alkyl, aryl, alkylthio, arylthio or acylaminoalkylthio group, $R^2$ and $R^3$ are each independently an alkoxycarbonyl or cyano group or a group of the formula $COR^1$ ($R^1$ being as above defined), $R^3$ may further be an alkyl or aryl group, and $R^4$ is a hydrogen atom, an alkyl, hydroxyalkyl or aryl group or an amino group which may optionally be protected, or a salt or ester thereof, which comprises reacting a compound of the formula (VII):

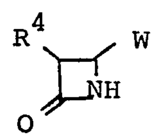

(VII),

wherein W is an acyloxy, sulfonyl or azido group or a halogen atom, and $R^4$ is as above defined

with a compound of the formula (VIII):

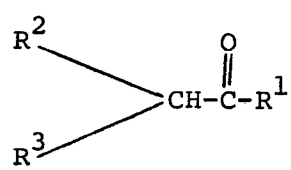

(VIII)

(wherein $R^1$, $R^2$ and $R^3$ are as above defined).

10. An antimicrobial agent which contains as an active ingredient an effective dose of a carba-2-penem compound having the formula (I):

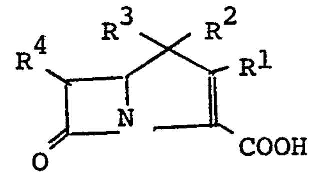

(I),

wherein $R^1$ is an alkyl, aryl, alkylthio, arylthio or acylaminoalkylthio group, $R^2$ and $R^3$ are each independently an alkoxycarbonyl or cyano group or a group of the formula $COR^1$ ($R^1$ being as above defined), $R^3$ may further be an alkyl or aryl group, and $R^4$ is a hydrogen atom, an alkyl, hydroxyalkyl or aryl group or an amino group which may optionally be protected, or a salt or ester thereof, together with a suitable carrier or carriers.

11. An antimicrobial agent which contains a beta-lactamase inhibiter an effective amount of a carba-2-penem compound having the formula (I):

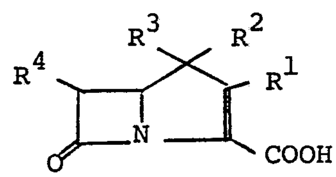

(I),

wherein $R^1$ is an alkyl, aryl, alkylthio, arylthio or acylaminoalkylthio group, $R^2$ and $R^3$ are each independently an alkoxycarbonyl or cyano group or a group of the formula $COR^1$ ($R^1$ being as above defined), $R^3$ may further be an alkyl or aryl group, and $R^4$ is a hydrogen atom, an alkyl, hydroxyalkyl or aryl group or an amino group which may optionally be protected, or a salt or ester thereof, in combination with beta-lactam type antibiotic(s), together with a suitable carrier or carriers.

12. A compound (I) or (III) as defined in any of Claims 1 to 7, or a composition as defined in Claim 10 or 11, for use as an active ingredient of an antimicrobial agent, or for use as a beta-lactamase inhibiter in combination with one or more beta-lactam type antibiotics, or for use as a drug in humans or domestic animals.

CLAIMS

1. A method of producing novel carba-2-penem compounds of the formula (III):

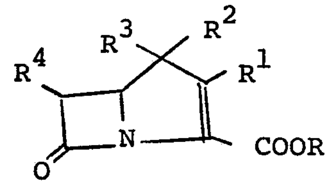

(III)

wherein $R^1$ is an alkyl, aryl, alkylthio, arylthio or acylaminoalkylthio group, $R^2$ and $R^3$ are each independently an alkoxycarbonyl or cyano group or a group of the formula $COR^1$ ($R^1$ being as above defined), $R^3$ may further be an alkyl or aryl group, $R^4$ is a hydrogen atom, an alkyl, hydroxyalkyl or aryl group or an amino group which may optionally be protected, and COOR is a protected carboxyl group,

which comprises subjecting a compound of the formula (II):

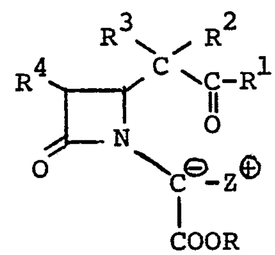

(II),

wherein Z is a phosphonio or phosphono group, and the symbols COOR and $R^1$ to $R^4$ have the above meanings,

to ring closure.

2. A method according to Claim 1 for producing a novel carba-2-penem compound having the formula (I):

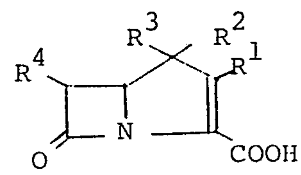

(I),

wherein $R^1$ is an alkyl, aryl, alkylthio, arylthio or acylaminoalkylthio group, $R^2$ and $R^3$ are each independently an alkoxycarbonyl or cyano group or a group of the formula $COR^1$ ($R^1$ being as above defined), $R^3$ may further be an alkyl or aryl group, and $R^4$ is a hydrogen atom, an alkyl, hydroxyalkyl, or aryl group or an amino group, which may optionally be protected,
or a salt or ester thereof, wherein said COOR group in said formula (III) is optionally converted to COOH by a method known per se.

3. A method as claimed in Claim 2, wherein said group R is selected from halo- and polyhalo-lower alkyl, aralkyl, benzhydryl and nitrobenzyl.

4. A method as claimed in Claim 1, wherein $R^1$ is a $C_{1-6}$ alkyl, phenyl, naphthyl, $C_{1-6}$ alkylthio, phenylthio, naphthylthio or $C_{1-6}$ acylamino-$C_{1-6}$ alkylthio group, $R^2$ and $R^3$ are each independently a $C_{1-6}$ alkoxycarbonyl or cyano group or a group of the formula $COR^1$ ($R^1$ being as above defined), $R^3$ may further be a $C_{1-6}$ alkyl, phenyl or naphthyl group, and $R^4$ is a hydrogen atom, a $C_{1-6}$ alkyl, hydroxy-$C_{1-6}$-alkyl, phenyl or naphthyl or an amino group which may optionally be protected, said $C_{1-6}$ alkyl, phenyl and naphthyl being unsubstituted or substituted with one or more of a $C_{1-2}$ alkoxy-$C_{1-2}$ alkyl, a $C_{1-2}$ alkoxyphenyl, a $C_{1-2}$ alkoxynaphthyl, a halogeno-$C_{1-2}$ alkyl, a halogenophenyl and a halogenonaphthyl, further said phenyl and naphthyl being unsubstituted or substituted with a $C_{1-6}$ alkyl.

5. A method as claimed in Claim 4, wherein a protective group of the protected amino group represented by $R^4$ is a $C_{1-6}$ alkylcarbonyl which may be substituted with a halogen, $C_{1-6}$ alkoxycarbonyl which may be substituted with a halogen, a $C_{1-6}$ alkoxy - $C_{1-6}$ alkylcarbonyl, a $C_{7-19}$ aralkyloxycarbonyl, a $C_{7-19}$ aralkyl or tetrahydropyranyl group.

6. A carba-2-penem compound as claimed in any of Claims 1 to 3, wherein $R^1$ is a $C_{1-4}$ alkyl, phenyl, $C_{1-4}$ alkylthio, phenylthio or a acetylamino-$C_{1-4}$ alkylthio, $R^2$ is a $C_{1-4}$ alkoxycarbonyl, cyano or a $C_{1-4}$ alkylcarbonyl, $R^3$ is a $C_{1-4}$ alkyl, phenyl, a $C_{1-4}$ alkoxycarbonyl or a $C_{1-4}$ alkylcarbonyl, and $R^4$ is a hydrogen atom, $C_{1-4}$ alkyl or phenyl.

7. A method as claimed in any of Claims 1 to 3, wherein $R^1$ is a $C_{1-4}$ alkyl, phenyl or a $C_{1-4}$ alkylthio, $R^2$ is a $C_{1-4}$ alkoxycarbonyl, a $C_{1-4}$ alkylcarbonyl or cyano, $R^3$ is a $C_{1-4}$ alkoxycarbonyl, a $C_{1-4}$ alkylcarbonyl, a $C_{1-4}$ alkyl or phenyl, and $R^4$ is a hydrogen atom, a $C_{1-4}$ alkyl or a hydroxyl-$C_{1-4}$ alkyl.

8. A method of producing a novel compound of the formula (VI)

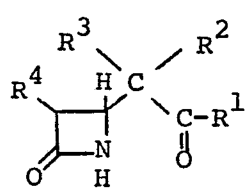

(VI),

wherein $R^1$ is an alkyl, aryl, alkylthio, arylthio or acylaminoalkylthio group, $R^2$ and $R^3$ are each independently an alkoxycarbonyl or cyano group or a group of the formula $COR^1$ ($R^1$ being as above defined), $R^3$ may further be an alkyl or aryl group, and $R^4$ is a hydrogen atom, an alkyl, hydroxyalkyl or aryl group or an amino group which may optionally be protected, or a salt or ester thereof, which comprises reacting a compound of the formula (VII):

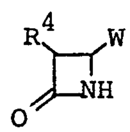

(VII),

wherein W is an acyloxy, sulfonyl or azido group or a halogen atom, and $R^4$ is as above defined

with a compound of the formula (VIII):

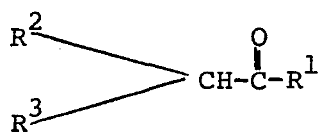

(VIII),

wherein $R^1$, $R^2$ and $R^3$ are as above defined.

9. A compound of the formula (I) or (III) as defined in any of Claims 1 to 7 or a composition containing an effective dose thereof as an active ingredient, for use as an antimicrobial agent, or as a drug in humans or domestic animals.

10. A compound of the formula (I) or (III) as defined in any of Claims 1 to 7, or a composition containing an effective amount thereof for use as a beta-lactamase inhibiter, in combination with one or more beta-lactam type antibiotics.